(19) Europäisches Patentamt
European Patent Office
Office européen des brevets

(11) **EP 3 324 320 A1**

(12) **EUROPEAN PATENT APPLICATION**

(43) Date of publication:
**23.05.2018 Bulletin 2018/21**

(51) Int Cl.:
*G06F 19/18* (2011.01)    *A61B 5/00* (2006.01)

(21) Application number: **17202813.6**

(22) Date of filing: **21.11.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **21.11.2016 US 201662424719 P**
**31.10.2017 US 201715799081**

(71) Applicant: **Johnson & Johnson Vision Care, Inc.**
**Jacksonville, FL 32256 (US)**

(72) Inventors:
- **FLITSCH, Frederick A.**
  **New Windsor, NY New York 12553 (US)**
- **PUGH, Randall B.**
  **Jacksonville, FL Florida 32256 (US)**

(74) Representative: **Carpmaels & Ransford LLP**
**One Southampton Row**
**London WC1B 5HA (GB)**

(54) **BIOMEDICAL SENSING METHODS AND APPARATUS FOR THE DETECTION AND PREVENTION OF LUNG CANCER STATES**

(57) The present invention relates to methods and apparatus to monitor and potentially prevent occurrences of lung cancer in patients. The invention includes apparatus and methods to determine a patient's pre-disposition or propensity to acquire lung cancer. Also included are apparatus and methods to monitor for patient biomarkers and for environmental agents, to warn and to take action to reduce exposure to environmental agents and to increase patient screening for the occurrence of lung cancer.

**EP 3 324 320 A1**

**Description**

**CROSS-REFRENCE TO RELATED APPLICATIONS**

This patent application claims the benefit of United States Provisional Patent Application No. 62/424,719 filed November 21, 2016. The contents are hereby incorporated by reference.

**BACKGROUND OF THE INVENTION**

1. Field of the Invention

The present invention relates to methods and apparatus to monitor and potentially prevent occurrences of lung cancer in patients. The field includes apparatus and methods to determine a patient's pre-disposition or propensity to acquire lung cancer.

2. Discussion of the Related Art

Cancer is not a single disease, but rather a collection of related diseases that can start essentially anywhere in the body. Common amongst all types of cancer is that the body's cells begin to divide without stopping, proliferating and potentially spreading into surrounding tissues. In the normal course of events, cells grow and divide to form new cells as required by the body and when they become damaged or old, they die and new cells replace the damaged or old cells; however, cancer interrupts this process. With cancer, the cells become abnormal, and cells that should die do not and new cells form when they are not needed. These new cells can reproduce or proliferate without stopping and may form growths called tumors.

Cancerous tumors are malignant, which means they can spread into or invade surrounding health tissue. In addition, cancer cells can break off and travel to remote areas in the body through blood or in the lymph system. Benign tumors, unlike malignant tumors, do not spread or invade surrounding tissue; however, they may grow large and cause damage. Both malignant and benign tumors may be removed or treated. Malignant tumors tend to grow back whereas benign tumors can grow back, but are much less likely to do so.

Cancer is a genetic disease in that it is caused by changes in the genes that control the ways cells function, especially in how they grow and divide. Genetic changes that cause cancer may be inherited or they may arise over an individual's lifetime as a result of errors that occur as cells divide or because of damage to DNA caused by certain environmental exposure, for example, industrial/commercial chemicals and ultraviolet light. The genetic changes that may cause cancer tend to affect three types of genes; namely proto-oncogenes which are involved in normal cell growth and division, tumor suppressor genes which are also involved in controlling cell growth and division, and DNA repair genes which, as the name implies, are involved in repairing damaged DNA.

More than one-hundred distinct types of cancer have been identified. The type of cancer may be named for the organ or tissue where the cancers arise, for example, lung cancer, or the type of cell that formed them, for example squamous cell cancer. Cancer, unfortunately, is a leading cause of death both in the United States and world-wide. According to the World Health Organization, the number of new cancer cases will rise to twenty-five (25) million per year over the next two decades.

Lung cancer is one of the most common cancers today. According to the World Cancer Report 2014 from the World Health Organization, lung cancer occurred in 14 million people and resulted in 8.8 million deaths world-wide, making it the most common cause of cancer-related death in men and the second most common cause of cancer-related death in women. Lung cancer or lung carcinoma is a malignant lung tumor that if left untreated can metastasize into neighboring tissues and organs. The majority of lung cancer is caused by long-term tobacco smoking; however, about 10 to 15 percent of lung cancer cases are not tobacco related. These non-tobacco cases are most often caused by a combination of genetic factors and exposure to certain environmental conditions, including radon gas, asbestos, second-hand tobacco smoke, other forms of air pollution, and other agents. The chance of surviving lung cancer as well as other forms of lung cancer depends on early detection and treatment.

As set forth above, cancer may be caused by a combination of genetic factors and environmental factors. Accordingly, individuals with a genetic predisposition towards cancer, for example lung cancer, should preferably be monitored for exposure to environmental factors that are known to cause or trigger this genetic cascade. Sensors or sensor arrays may be adapted to detect analytes in the environment and alert individuals of the potential risk of exposure as well as to detect analytes produced by cancer cells for early detection. The sensor array would preferably be portable and configured to sample both analytes in the environment and produced by individuals, for example, analyze breath and/or other bodily emissions. It would be desired to develop protocols that could be based on patient pre-disposition to lung cancer and would associate appropriate levels of environmental monitoring and patient screening contingent upon the genetically determined pre-disposition to lung cancer.

**SUMMARY OF THE INVENTION**

Accordingly, methods and apparatus to monitor the environment of genetically pre-dispositioned patients and warn them to take action are disclosed herein. Additionally, methods and apparatus to sense the emissions, biofluids, biome and biometrics of the pre-dispositioned patients are also disclosed. Patients who receive a warning may take action including additional periodic screenings, avoiding locations with the potential for undesired exposure to levels of environmental agents which may be correlated with increased lung cancer risk for pre-disposed patients. In some examples, the action that the patient takes may include the donning of protective apparel such as masks with adsorbent material or filters. The apparatus may sense the level of exposure of the patient and record the results in a database. In some examples, the Patient may wear the monitoring apparatus to a doctor visit with the data logged on the device. The historical record of exposure may be used by medical professionals to determine the level of screening to be performed on the patient such as in a non-limiting example computed tomography (CT) x-ray scanning of the lungs of the patient.

In an example, a system for reducing a potential risk of obtaining cancer may be formed comprising a number of pieces of apparatus. In some examples, the system may include a thermal cycler for performing genetic amplification, wherein the result of the genetic amplification is used in determining a sequence of DNA of a patient to determine variation at target regions of a patient's genome. The system may include a first sensing device wherein the first sensing device is installed in a location of the home of the patient. The system may include a second sensing device wherein the second sensing device is installed in a location of the work location of the patient. The system may include a processing system, wherein the processing system performs a first algorithm which processes stored data originating from the first sensing device and originating from the second sensing device and determines a status to a threshold level, wherein the threshold level is determined based on a result of the determined variation at target regions of the patient's genome. In some examples, a third sensing device may be associated with the patient themselves, such as a sensing apparatus attached to or incorporated into a smartphone or other wearable device. The system may also include a feedback device, wherein the feedback device communicates a signal to the patient based on the status to the alarm level. In alternative embodiments, the system may be implemented as a stand-alone or self-contained device, such as a device that may be worn or carried.

This composition of the system may have various flexibility in the nature of the components. In an example, the system may include examples where the first sensing device is a non-specific chemical sensor. In an example, the system may include examples where a plurality of elements in the non-specific chemical sensor respond to an exposure to a chemical in an environment surrounding the non-specific chemical sensor in a pattern of responses which are correlated to a first chemical suspected of being related to an increased risk for developing lung cancer in the patient. In some examples, the monitoring apparatus may comprise a plurality of non-specific chemical sensors which are connected to a neural network for pattern recognition and calculation in real time. In still further examples, small deep-neural nets, which may comprise as little as a half a megabyte of memory may be used for an embedded system comprising a deep-neural net to recognize the patterns of chemical arrays in real time and detect multiple component systems.

In an example, the system may include examples where the first chemical is radon. In an example, the system may include examples where the first chemical is bis(chloromethyl) ether. In an example, the system may include examples where the first chemical is of a family ofpolycyclic aromatic hydrocarbons. In an example, the system may include examples where the non-specific chemical sensor comprises a polymer film doped with chemical species that modulate the absorbent properties of a surface of the polymer film. In an example, the system may include examples where the non-specific chemical sensor comprises crystals whose natural oscillation frequency is modulated by absorbance of materials onto surfaces or onto films applied to surfaces of the crystal. In an example, the system may include examples where the non-specific chemical sensor detects biomarkers of lung cancer in the breath of the patient.

In one embodiment, the system may include examples where the first sensing device is a particulate sensing device. In an example, the system may include examples where the first sensing device senses at least a first biological agent. In an example, the system may include examples where the biological agent is *Mycobacterium tuberculosis*.

In another embodiment, the system may include examples additionally comprising a personal sensing device that senses the immediate environment surrounding the patient. In an example, the personal sensing device senses radioactivity. In an example, personal sensing may include chemical exposure sensing. In an example, the personal sensing may include particulate exposure sensing. In an example, the personal sensing may include blood sensing. In an example, the personal sensing may include sensing of a biometric. The biometric may be inhalation rate or the patient's pulse in some examples.

In some embodiments, the personal sensor may be located within the patient's lung. This personal sensor may sense chemical markers. In some examples, this personal sensor may sense exposure to environmental chemicals. In some other examples, this personal sensor may sense radioactivity.

In some examples a system may be created for detecting a form of cancer where the system include a means for obtaining genetic data of a patient to determine variation at target locations of the patient's genome, wherein the variations are related to an increased risk of having the form of cancer. The system may also include a first database record, wherein the first database record contains records of familial health history. In some examples, the database records of familial health history may be dynamically updated. The system may also include a second database record, wherein the second database record contains one or more of a null dataset and a record of an environmental exposure of the patient. Hereto the database record of environmental exposure may be dynamically updated based on data obtained by the system as well as data communicated to the system over networks. The system may include a first sensor system, wherein the first sensor system is configured to sense biomarkers related to a cancer state, wherein the biomarkers emanate from the user. The system may also include a second sensor system, wherein the second sensor system is configured to sense the local environment of the user for one or more of particulate matter and chemical pollutants. These elements of the system may also be linked to an included processing system. The processing system implements at least one of: a first algorithm and a second algorithm. The first algorithm may include an example which processes data from the first sensor system to compare a first sensor system result to a threshold level, wherein the threshold level is determined based on one or more of a result of the determined variation at target locations of the patient's genome, processing of data within the first database record, and processing of data within the second database record. The second algorithm may process data from the second sensor to determine environmental conditions at the patient's location. The system may also include a feedback device configured to communicate a signal to the patient based on the results of processing by the processing system. Thus when the system detects conditions of interest or concern to a user a communication of the conditions may be made with the user.

Implementations may include various methods. In an example, a method of reducing a risk of acquiring lung cancer of a patient predisposed to acquiring lung cancer may include various steps. The method may include determining the predisposition of the patient to acquiring lung cancer by analyzing the patient's genome. The method may include installing a first sensing device wherein the first sensing device is installed in a location of the home of the patient. The method may include installing a second sensing device wherein the second sensing device is installed in a location of the work location of the patient; and the method may include installing a processing system, wherein the processing system performs a first algorithm which processes stored data originating from the first sensing device and originating from the second sensing device and determines a status to a threshold level, wherein the threshold level is determined based on a result of the determined variation at target regions of the patient's genome. The method may also include installing a feedback device, wherein the feedback device communicates a signal to the patient based on the status to the alarm level. The method may include monitoring a first environmental status at the home of the patient with the first sensing device. The method may include monitoring a second environmental status at the work location of the patient with the second sensing device. The method may include detecting a signal from one or more of the first sensing device or the second sensing device, wherein the signal exceeds the threshold level. The method may include communicating a signal to the patient based on the detected signal. And, the method may include taking an action to reduce the exposure of the patient to one or more of the first environmental status or the second environmental status. The various method steps may include the various options as have been described in relationship to the system previously.

## BRIEF DESCRIPTION OF THE DRAWINGS

The foregoing and other features and advantages of the invention will be apparent from the following, more particular description of preferred embodiments of the invention, as illustrated in the accompanying drawings.

Fig. 1 illustrates an exemplary model of pre-disposition to lung cancer and exposure to environmental agents and the combined effects on the chance to develop lung cancer in accordance with the present invention.

Fig. 2A illustrates exemplary environmental sensing location types in accordance with the present invention.

Fig. 2B illustrates exemplary individual sensing modalities in accordance with the present invention.

Fig. 3A illustrates an exemplary multichannel broad-spectrum sensing device in accordance with the present invention.

Fig. 3B illustrates exemplary results that individual elements of a device from Fig. 3A may produce upon exposure to environmental agents in accordance with the present invention.

Fig. 4 illustrates an exemplary particulate-sensing device in accordance with the present invention.

Fig. 5A and 5B illustrate an exemplary colorimetric device for sensing biological agents in accordance with the present invention.

Figs. 6A and 6B illustrate an exemplary sensing device for biological agents in accordance with the present invention.

Fig. 7A illustrates an exemplary spectral technique for generating an absorption spectrum in accordance with the present invention.

Figs. 7B, 7C and 7D illustrate exemplary devices that may be useful for performing the technique of Fig. 7A in accordance with the present invention.

Figs. 8A-8E illustrate exemplary personal sensing devices that may be useful for detecting biomarkers of patients related to lung cancer in accordance with the present invention.

Fig. 9 illustrates exemplary method steps that may be useful in treatment and prevention of lung cancer in patients in accordance with the present invention.

Fig. 10 illustrates additional exemplary method steps that may be useful in treatment and prevention of lung cancer in patients in accordance with the present invention.

Fig. 11 illustrates exemplary method steps that may be useful in treatment and prevention of lung cancer in patients in accordance with the present invention.

## DETAILED DESCRIPTION OF THE INVENTION

Although shown and described in what is believed to be the most practical and preferred embodiments, it is apparent that departures from specific designs and methods described and shown will suggest themselves to those skilled in the art and may be used without departing from the spirit and scope of the invention. The present invention is not restricted to the particular constructions described and illustrated, but should be constructed to cohere with all modifications that may fall within the scope of the appended claims.

Methods of forming and using sensing elements to diagnose and treat lung cancer states and potentially delay or prevent their development are disclosed in this application. A simplified model of the general nature of the acquisition of lung cancer in a patient may be that the likelihood of acquisition is a function of an individual's root propensity for a type of lung cancer which intersects with an exposure to an environmental contributor. Thus, there may be examples of individuals who have a low propensity to obtaining a form of lung cancer who are exposed to high levels of an environmental contributor, such as a volatile chemical, and never acquire a lung cancer. It is possible that other individuals who have a high propensity to acquire a type of lung cancer may get that lung cancer while seemingly having nearly no exposure to particular environmental contributors.

Advancements in chemical sensing technology and environmental sensing technology, particularly advancements that have gone on over the past decade and continue today, when coupled with advances in genomic mapping and the understanding of genetic contributions to various forms of lung cancer allow for novel treatment methods which are preventative, diagnostic and therapeutic.

Glossary

In the description and claims below, various terms may be used for which the following definitions will apply:

"Biocompatible" as used herein refers to a material or device that performs with an appropriate host response in a specific application. For example, a biocompatible device does not have toxic or injurious effects on biological systems.

"Coating" as used herein refers to a deposit of material in thin forms. In some uses, the term will refer to a thin deposit that substantially covers the surface of a substrate it is formed upon. In other more specialized uses, the term may be used to describe small thin deposits in smaller regions of the surface.

"Functionalized" as used herein refers to making a layer or device able to perform a function including, for example, energization, activation, and/or control.

Genetic Predisposition to Lung Cancer and Environmental Factors

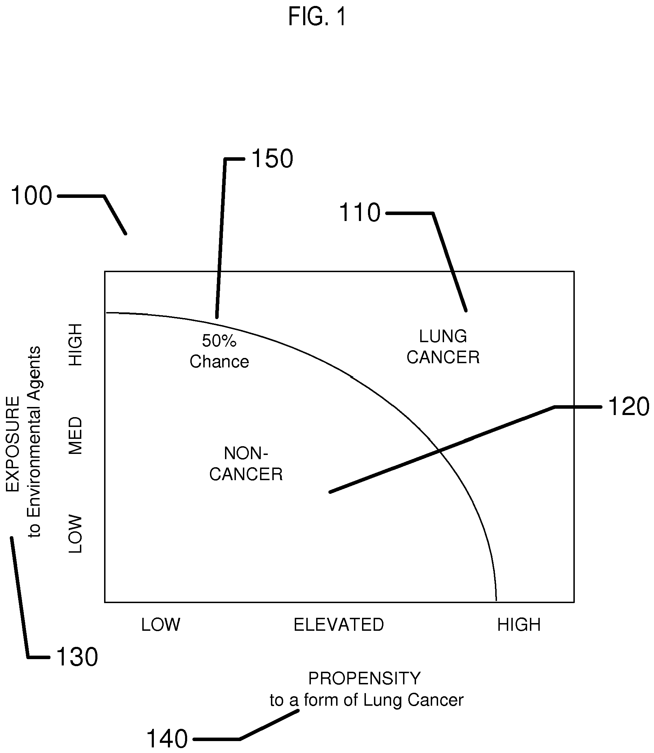

Studies have shown that significant incidence of occurrence of lung cancer may be modeled as an interaction between an individual's genetic predisposition with the amount and nature of that individual's exposure to various environmental factors. For example, one of the largest current environmental factors related to many individual's acquiring of lung cancer is their smoking of tobacco products. Yet, anecdotal evidence is commonly known that there are cases of individuals who smoked large amounts of tobacco products during their lives but never acquired lung cancer as well as individuals who never smoked themselves but acquired lung cancer nevertheless. Therefore, for some portions of acquired lung cancer, a model 100 such as that depicted in Fig. 1 may be apropos. As Fig.1 illustrates, an individual's chance of acquiring lung cancer may be plotted as a line, for example, at an illustrative 50% chance level 150 across dimensions of exposure to environmental agents 130 versus an individual's propensity to develop lung cancer 140. Where movement above the line may increase the chance of lung cancer 110 and movement below the line may increase chance of non-cancer 120. Thus, as development of knowledge about how genetic mutations and inherited genetic anomalies may create predisposition to lung cancer, strategies and equipment may be developed which may allow individuals to monitor their exposure to various environmental agents. Thus, when a patient's history and makeup lead a physician to perform genetic testing to assess his genetic predisposition, a patient may learn he has an elevated or even a high level of "propensity" or predisposition to acquisition of lung cancer. In the model 100, a patient with an elevated or high propensity will have a profile for his or her chance of developing lung cancer as illustrated at the right hand side of the chart. Thus, if a patient can minimize or eliminate his or her exposure to important environmental agents, that patient may significantly improve his chances of not acquiring lung cancer. Thus, investment into sensing equipment and the effort to control sensed exposures may create significant improvements in outcomes.

There may be numerous variations in certain known genes that relate to the determination of genetic propensity. However, this knowledge is ever improving, and the spirit of the discussion herein is meant to embrace inherently flexible adoption of new knowledge as it becomes developed. The same flexibility may be accorded to the development of knowledge about important environmental agents that may be important for a patient with elevated or high propensity to avoid. Nevertheless, there may be important genetic variations that are currently suspected as increasing genetic propensity for lung cancer. For example, one of the important areas of genetic variation that has been focused upon is polymorphic variation at genetic location 5p15.33 (TERT/CLPTM1L), at genetic location 6p21.33 (BAT3/MSH5) and at genetic location 15q25.1 (CHRNA5/CHRNA3/CHRNB4) in patients of European background. Additionally, single-nucleotide polymorphisms (SNPs) at genetic location 22q12 (10,11) and the 15q15.2 locus containing the TP53-binding protein 1 gene have been associated with lung cancer risk. In patients of Asian background there may be three additional susceptibility regions of focus at genetic locations 13q12.12, 22q12.2 (15) and 3q28 (16). Whereas tobacco smoking is the predominant risk factor for lung cancer, there are several lines of evidence to suggest that genetic susceptibility modulates this risk. Investigators working on the European Prospective Investigation into Cancer and Nutrition (EPIC) have identified several intriguing loci with more common population frequencies. At the previously mentioned chromosome 15q25, is a locus that contains several nicotinic acetylcholine receptors. Some results of these studies have shown variants at the previously mentioned 5p15.33, a region containing the hTERT and CLPTM1L genes3, hMSH5 in the chromosome 6 MHC region1, RAD52, and CDNK2A4.

In a high proportion of these mentioned genetic loci are varients that also show relevance to a particular histology (MHC, CDKN2A, RAD52, hTERT), which may suggest that for lung cancer, histological subtype may be important. As mentioned, there may be other genes and variations that are known or yet to be known that may be probed for with genetic testing to assess an individual's propensity to acquire lung cancer. Methods may probe for these various genetic variations and, in a semi-quantitative manner, the results may be used to classify a patient's risk profile into numerous strata, such as low, elevated and high propensity for pre-disposition to lung cancer. Any of the commercially and scientifically viable means to determine genetic variation in targeted gene loci may be applied to samples of genetic matter from patients to determine a relative level of pre-disposition risk to lung cancer.

A system related to the present invention may include devices that may measure or monitor genomic data for disposition risk to lung cancer. A sample from a patient may be processed with a thermal cycler for performing genetic amplification, wherein the result of the genetic amplification is used in subsequent steps for determining a sequence of DNA of a patient to determine variation at target regions of a patient's genome. Important regions to screen for mutation may be assessed to determine the risk.

In similar models, devices which are used to monitor a patient's environment, such as a smart phone equipped with monitoring devices, may in turn include an application which performs the monitoring, and also includes a capability to access data across an internet connection. In some examples, a patient may have submitted a sample of his genetic material to a commercial organization who sequences various locations of the patient's genome and records the results in a database. The application may have access to the data or may receive communication from the commercial organization which accesses the data and passes identification of genetic modifications of interest to the application on the smart device, which may alter or supplement its analysis conditions based upon the identified genetic modifications. In some examples, the commercial organization or another organizations database may include aspects of an individual's family health history. If the Patient activates appropriate means of "opting-in" to receiving such family health and genomic history, the smartphone application may also receive such data in concentrating its analysis. In an example, a record of a family member may indicate an occurrence of a particular type of cancer which population analysis may be correlated to sensitivity to specific environmental agents and conditions which may be monitored by the monitoring apparatus. Given a specific sensitivity resources of the analytical device may be prioritized and/or dedicated to the analysis of the particular environmental agent or condition.

Similarly, as depicted in the exemplary model of Fig. 1, there are numerous general environmental aspects and agents that may be associated with increased chance of acquisition of lung cancer. As the knowledge of important genetic variations to use to gage propensity may be increased, the ability to identify even further important environmental agents may increase as well. Currently, it may be widely accepted that tobacco smoke is an environmental agent of acute importance to lung cancer that may be important if the patient is a primary smoker as well as an individual exposed to second-hand smoke.

Other environmental agents may include exposure to radon gas. Radon is produced by the natural breakdown of uranium in soil, rock and water that may eventually become part of the air a patient may be exposed to. Radon is of particular importance because of its prevalence in many areas of the world. For example, in homes having basements, radon contamination over a long period of time may pose a health risk.

In some examples, asbestos and other fibrous silicon containing compounds may be important environmental agents to track. Other materials that may be common in the workplace include elemental compounds containing arsenic, beryllium, cadmium, chromium and nickel. Many of these environmental agents can be compounded in importance when they occur in multiple types of exposure.

Air pollutants related to the combustion of diesel and other fossil fuels as well as other chemicals such as bis(chloromethyl) ether and polycyclic aromatic hydrocarbons may also be important environmental agents that increase risk of developing lung cancer.

In some examples, infectious agents such as tuberculosis may be important environment agents of exposure which can increase the chance of acquisition of lung cancer.

Exposure to small particulate matter of many different elemental compositions may also be important environmental exposure risks to patients. This particulate matter, which shows increased risk factors for lung cancer may be summarized into at least two buckets. The PM2.5 (particulates found in the air whose size is 2.5 microns or less) and PM10 (particulates found in air whose size is 10 microns to 2.5 microns) are measured parameters that may be correlated to risk of lung cancer development. The risk factors may be similar for different types of lung cancer such as squamous cell and adenocarcinoma, and the risk factors may be modulated based on the life history of patients relating to whether they are a smoker, a past smoker or someone who has never smoked.

There may be numerous agents within various types of smoke that relate to risk of lung cancer acquisition. Amongst the components of various types of smokes, particulates that are even finer than the micron scale may be called nanoparticles and may be important risk agents. The development of commercial products with engineered nanoparticles is an emerging product type which may change aspects related to occupational and personal exposure over the coming decades. Numerous types of sensors may be able to monitor for particulates and for nano-scale particulates, as will be discussed in later sections. A unique feature of nanoparticles is that they have a very large surface area to mass ratio. Thus, effects of exposure to them might be more related to surface area than mass.

Although there may be no epidemiological evidence from human studies which conclusively demonstrates a lung cancer risk association with nanoparticle exposure, early evidence from experimental toxicity studies may suggest potential cause for concern. Thus, methods of monitoring such materials for patients with high pre-disposition to lung cancer may be prudent.

Exemplary Sensing Devices

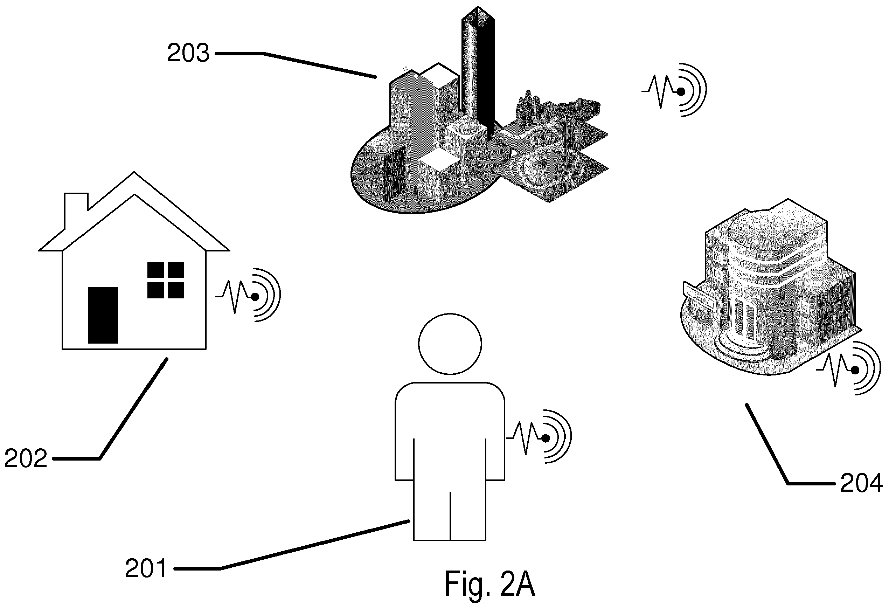

As has been mentioned, it may be advantageous to monitor air related to environments that people, or in particular people with high predisposition to lung cancer, may breathe. Referring to Fig. 2A a summary of the different types of environments that may be monitored is shown in exemplary form.

A person may spend an appreciable portion of their time in residence 202. Thus, various rooms may be equipped with various sensing equipment such as particulate monitors, specific chemical monitors, radioactivity monitors such as for radon, biological sensors, and broad-spectrum monitors that may be sensitive to various types of chemical and particulate forms. These types of sensors are described in more detailed form in subsequent sections. Different rooms in a house may have one or more of these types of sensors located. Bedrooms, kitchens, and living rooms may provide focal locations for sensing, but other locations such as in the basement and/or garage may also emerge in importance.

A person may spend time in their outside environment 203. An outside environment 203 may include confined spaces that are not at a home or workplace and may, therefore, include the interior space of an automobile for example. As well, external environments such as parks, walking paths, yards, decks and the like may be frequented locations where the air quality may be important for patients with a pre-disposition to lung cancer. Municipalities, and state agencies may establish regional monitoring stations for sensing of various kinds, including particulate matter, and the chemical nature of air pollution. In some examples, the real-time or near-real-time sensing output of such monitoring may be communicated via delivery means such as the internet and wireless communication. So-called "Smart Cities" may have a plurality of sensors deployed in urban areas where the sensors act as Internet of Things (IoT) devices which have a small power source which may be rechargeable, the sensing means, and a communication capability to interface with the internet or other networks. In some examples, a method according to the present invention may include apparatus and algorithms that access internet distributed databases that contain quality measure related to external air environments. The results of a number of sensors, which may be geographically separated on the scale of a human being may none the less be used to interpolate regionally averaged exposure estimates to a variety of environmental agents that may be of concern to lung cancer patients or patients with elevated or high pre-disposition to lung cancer.

Communications between a plurality of sensors may be able to be formed into different types of network topologies. In some examples, a plurality of mostly stationary sensors with communication capabilities may be arranged into wireless mesh networks. In some examples, stationary network nodes may interact with wireless nodes to form a complex network topology. The various distributed sensors may be useful for monitoring environmental conditions while at the same time participate in forming, amongst themselves, robust fault tolerant networks where data communication can be facilitated and made very efficient. Thus a system of environmental monitoring sensor systems, distributed to roughly stationary and reasonably dense locations may form a meshed network base that may support other mobile nodes that enter into the mesh environment. Thus patients who wear monitoring devices that can link to the local environmental sensing mesh network may be able to link to the sensing systems while also providing data themselves. Systems distributed amongst numerous patients/users may likewise participate or assemble with wireless mesh form networks.

External environments that are themselves contained, such as automobiles, as mentioned previously may have internal sensors that monitor the quality of the air within the contained environment. In some examples, automotive devices may have smart devices incorporated that may interface with the internet and access information related to the environmental air quality measured by sensing devices. The resulting database interpolations may be used to plan and execute routing decisions for pre-disposed patients as they are transported from one place to another. In some examples, they may be able to warn the patient that the terminus of the planned route is projected to be experiencing an increased level of an environmental agent of interest. Such information may be useful to change the planned transportation. In some examples, the patient may be delivered to a terminus via an internal parking garage for example. In other examples, the patient may not travel to the terminus entirely. And, in other examples, the patient may employ a number of air purification strategies such as particulate filters and chemical/absorbent material filters. In still further examples, the estimated exposure of the patient based on sensors in a closed environment or regional sensor networks may be associated in a database of the patient as an accumulated exposure estimate. In some examples, devices that can track the geolocation of individuals as they move from place to place may be useful in providing improved accuracy of regionally estimated exposures as a patient moves through a region.

Referring again to Fig 2A, an individual's place of work 204 may have monitoring systems deployed for the purpose of monitoring the airspace for all stakeholders at the location, or in some examples to monitor the air space for individuals with elevated pre-disposition to lung cancer. Buildings may be equipped with various sensing equipment such as particulate monitors, specific chemical monitors, radioactivity monitors such as for radon, biological sensors, and broad-spectrum monitors that may be sensitive to various types of chemical and particulate forms. As well, in work places there may be much higher likelihood of exposure for certain types of materials such a volatile organic carbon gasses, many forms of particulates and aerosolized metal containing compounds. Sensors that have been trained to detect the chemicals that are brought to the work place or that are likely byproducts of processes that are occurring in the work place may be specifically installed.

In nearly all of these environments and any others that may be on the interface between these types of environments, a patient may employ personalized sensing equipment 201. There may be numerous types of equipment that have sensors included in them and are associated with a user either by being a wearable sensor such as a smart watch or by being associated with an appliance that a user will tend to keep with them such as a cell phone, as non-limiting examples. Personal sensors may be equipped with various sensing equipment such as particulate monitors, specific chemical monitors, radioactivity monitors such as for radon, biological sensors, and broad-spectrum monitors that may be sensitive to various types of chemical and particulate forms. In some examples, a sensing device may include a number of these different sensing capabilities. In some other examples, a plurality of devices may be used to perform different sensing tasks. The personalized sensing equipment 201 may be chosen or "trained" for non-specific sensing equipment to monitor the near environment of the patient in a nearly real-time manner.

In some examples, the monitoring apparatus may comprise a plurality of non-specific chemical sensors which are connected to a neural network for pattern recognition and calculation in real time. Artificial neural networks (ANNs) or connectionist systems are computing systems inspired by the biological neural networks that constitute animal brains. Such systems learn (progressively improve their ability) to do tasks by considering examples, generally without task-specific programming. For example, in image recognition, they might learn to identify images that contain cats by analyzing example images that have been manually labeled as "cat" or "no cat" and using the analytic results to identify cats in other images. They have found most use in applications difficult to express with a traditional computer algorithm using rule-based programming. A neural network as used herein, which may also be called and artificial neural network when not involving biological neurons, is based on a collection of connected units called artificial neurons. The connections between the connected units may be called synapses and they can be used to transmit a signal from the first neuron to another neuron. The receiving neuron can process the signal(s) and then signal downstream neurons connected to it. Neurons may have "state" defined. The state may be represented by a value which may typically be represented between 0 and 1. As learning occurs with a system of synapses and neurons, the weighting factor that a neuron is given may vary. By varying the weighting, the system can adjust the strength of a signal that is communicated downstream.

A deep neural network is an artificial neural network with multiple hidden layers between the input and output layers. Deep neural networks can model complex non-linear relationships which may relate to the signals that non-specific sensor arrays generate particularly when combinations of analytes are present in the environment. A deep neural network may be used on arrays of sensors and may create architectures which generate models for the complex superposition of signal patterns for different combinations of analytes which improve in accuracy over time or "learn". The extra layers of a deep neural net may enable composition of features from lower layers, potentially modeling complex data with fewer units than a similarly performing shallow neural network. In still further examples, small deep-neural nets, which may comprise as little as a half a megabyte of memory may be used for an embedded system comprising a deep-neural net to recognize the patterns of chemical arrays in real time and detect multiple component systems in small form factors that can be incorporated in portable smart devices or biomedical devices. A sensing device with neural network processing components may be trained with standard mixtures of analytes to create an accurate sensing device that may function rapidly or may function in real time.

In some examples, the personalized sensing equipment 201 may be connected to devices capable of algorithmically processing the data associated with the sensing equipment. The devices may be enabled with warning trigger levels such that when a sensing device begins to see an elevated response to a monitored environmental agent it may communicate to the user a need to take an action. The means to communicate to the user may include any standard communication protocol, including in a non-limiting sense, an alarm, buzzer, vibration or visual screen display which alert the patient to an environmental condition that motivates him or her to take an action. The action may include evacuating the location or wearing a piece of protective apparel as non-limiting examples. In some examples, the connected device may maintain a database which records the various sensor outputs along with a time date stamp. The recorded data may be aggregated in various manners that may support decisions related to treatment of a patient. In some examples, evidence of elevated exposure events or integrated exposure amounts over time or combinational exposures (where more than one sensed exposure occurred simultaneously) may be used to elevate screening protocols such a x-ray analysis, blood monitoring or other such examples of medically appropriate screening for patients with a high or elevated pre-disposition to lung cancer.

Individual Sensing Examples

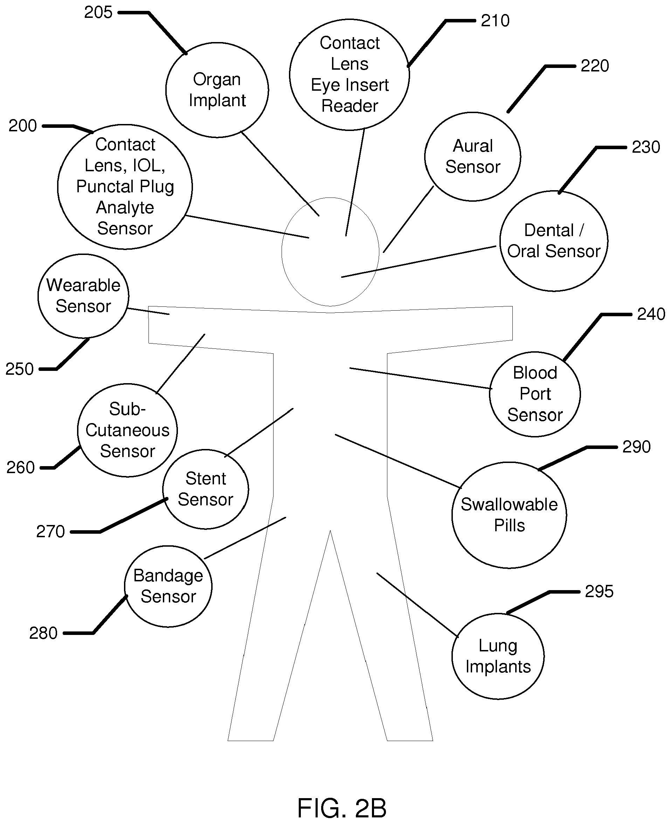

Referring to Fig. 2B, an exemplary illustration of numerous personalized sensing equipment examples is depicted. Included in the illustration are numerous devices that may be used to sense exposure to environmental agents. In some other devices, the purpose may be to monitor systems of the patient for evidence within the body of focused environmental agents, or metabolites of such focused environmental agents. In still further devices, the purpose of some of the monitor systems may be to sense the presence of biomarkers that may be correlated to the presence of a lung cancer disease state.

Some of the devices may operate in a manner that may indicate whether the patient has been exposed to pathogens of interest. In a non-limiting sense, Tuberculosis may be an example of a pathogen that has potential causative relationships with lung cancer in some infected individuals. Devices which can sense the presence of exposure to Tuberculosis, perhaps even before an infection has taken place may be of import. In some other examples, biometric information that may be indicative of a state of infection such as temperature, impaired or "noisy" breathing or other such metrics may be useful.

Still further devices may record biometric data that may be used to connect the physiological state of a patient during an exposure event. For example, pulse rate, breathing rate, breathing volume, and blood pressure may be useful biometrics to understanding the nature of uptake of an environmental agent that a patient may be exposed to.

There may be numerous types of biomedical related sensing techniques that may be used individually or in combinations to perform sensing of an individual consistent with the present invention. Referring again to Figure 2B, a summary of numerous exemplary types of biomedical devices may be found. Various ophthalmic devices 200, such as contact lenses, intraocular devices, punctal plugs, and the like may perform various sensing functions including analyzing analytes in the biofluids in the ocular environment. Tear fluid may contain chemicals and materials contributed from the body of the individual, but they also are significantly exposed to the environment of the individual and may contain a historical exposure record for a time period.

Contact lenses, 210 may also be used to read and quantify results from sensing devices that may be implanted into ocular tissue.

Implants into organs 205, may include brain implants, heart implants, pacemakers, and other implants that are implanted into organs of the user. These implants may be able to directly sense or indirectly sense a user's cellular tissue layer or a fluid contacting a user's cellular tissue layer. A specialized type is described with reference to a lung implant 295 as is discussed in greater detail subsequently.

In other examples, a biomedical sensing device may be an aural sensor 220. The aural sensor 220 may indirectly sense a biometric such as temperature as an infrared signal for example. The aural sensor 220 may also be able to quantify other biometrics such as blood oxygenation, analyte and bio-organism sensing and other such sensing. They also may be used to sense chemical emissions from the individual as well as environmental chemicals and materials in the space of the individual. Some biometrics may have potential diagnostic relevance in the detection of insults and challenges on the biome of an individual in his or her environment, or the biometrics may have diagnostic relevance to disease states.

A dental sensor 230 may be used to sense a variety of different types of biometric data. The sensor 230 may probe the fluids in the oral cavity for biomolecules and chemical species from food, and the biological fluids affected by environmental exposures of various kinds. The sensor 230 may also probe for indirect measurements of various types including in a non-limiting perspective pressures, temperatures, flows and sounds in the environment that may be directly or indirectly related to biometrics such as body temperatures, breathing rates, durations, strengths and the like. In addition, the dental sensor 230 may be utilized to sense biometric data during both inhalation and exhalation. Such a dental sensor may comprise non-specific sensors with neural net based control and/or analysis of data obtained from the sensor. In some examples a chemical separation membrane may allow volatile organic compounds to pass into a sensor while excluding aqueous based liquids. Polymers of Intrinsic Microporosity (PIM-1) based membranes made with designed combinations of block copolymers, which have micropores to allow permeation of gasses, may provide examples. Other examples may include polymers with intrinsic porosity such as polyacetylenes, fluorinated polymers, polynorbornanes, and polyimides as examples.

Vascular port sensors 240 may be used to sense various aspects within a blood stream. Some examples may include neuronal proteins or other hormones or antibodies that may be expressed during early stages of lung cancer and may be related to paraneoplastic syndrome. Any sensing example that interacts with bodily fluids that may contain these neuronal proteins or other hormones or antibodies that may be expressed during the early stages of lung cancer may operate in this manner and such examples are not meant to be limited to vascular port sensors 240. Other examples of sensing with a vascular port sensor 240 may be oxygen monitoring or other chemical monitoring. Other biometrics may be monitored at a vascular port such as blood pressure or pulse as non-limiting examples.

Some biometric sensors may be wearable sensors 250. A wearable sensor 250 may indirectly measure a variety of biometrics as well as chemicals and materials in the environment as well as those emitted by the individual. A well-known example is the sweat chloride assay for cystic fibrosis, a very serious lung disease. In this assay, sweat is collected from the individual and certain biomarkers are tested for. More specifically, the cystic fibrosis transmembrane conductance regulator (*CFTR*) gene located on the long arm of chromosome 7 (7q31.2) is examined. In some examples, the sensing element may be independent of any body tissue or body fluid of a user. Such a sensing element may monitor biometrics related to the user's body as a whole, or the chemical and particulate environment of the individual. In some examples, such devices may have geo-locating devices within them that may contain a record of where the individual has been over time. Such information may be married with data in external databases to infer levels of exposures of individuals. Other wearable sensors may directly or indirectly sense or probe a user's cellular tissue layer which may allow measurements of temperature, oxygenation, and chemical analysis of perspiration as non-limiting examples. The wearable sensors 250 may take the form of or be incorporated into clothing or jewelry in some examples. In other examples the wearable sensors 250 may attach to clothing or jewelry.

Various examples of biometric sensors may be incorporated into sub-cutaneous sensors 260 where a surgical procedure may place a biomedical device with sensors beneath a skin layer of a user. The sub-cutaneous sensor 260 may be sensitive with direct contact to tissue layers or to interstitial fluids. The sub-cutaneous sensor 260 may be able to analyze for various analytes, for example, with techniques described previously herein. Physical parameters may also be measured such as temperature, pressure and other such physically relevant biometric parameters.

Sensors may be incorporated into blood vessels or gastrointestinal stents of various kinds forming a stent sensor 270. The stent sensors 270 may therefore be able to perform sensing of various chemical species. Stent sensors 270 incorporated within blood vessels may be able to also characterize and measure physical parameters of various types. For example, a blood vessel form of stent sensor 270 may be able to measure pressures within the vessel during heart pumping cycles for a physiologically relevant determination of blood vessel pressure. There may be numerous manners that such a pressure sensor could function with small piezoelectric sensors, elastomeric sensors and other such sensors. There may be numerous physical parameters in addition to pressure that may be monitored directly within the blood stream. Chemicals including hormones as have been mentioned previously that may be relevant to a lung cancer state may be monitored as well.

A pill form biometric sensor, such as a swallowable pill 290 may be used to provide biometric feedback and may sense simple chemicals and bio-chemicals in an individual. In some examples, the swallowable pill may incorporate pharmaceutical components. In other examples, the swallowable pill 290 may simply contain biometric sensors of various kinds. The swallowable pill 290 may perform analyte measurements of the gastrointestinal fluids that it incorporates. Furthermore, the pills may provide central core temperature measurements as a non-limiting example of physical measurements that may be performed. The rate of movement of the pill through the user's digestive track may also provide additional information of biometric relevance. In some examples, analyte sensors may be able to provide measurements related to dietary consumption and nutritional aspects. Since inhaled chemicals and particulate matter traverses the mouth, throat and larynx, some record of exposure may proceed through saliva into the gastrointestinal tract where such a sensor may record their presence.

A bandage form sensor 280 may be used to perform biometric and environmental sensing. In some examples, the bandage form sensor 280 may be similar to a wearable sensor 250 and perform measurements upon chemicals in the skin environment including aspects of perspiration as well as incident chemical and material exposure analysis. The bandage form sensor 280 may also perform physical measurements. In some special examples, the bandage may be in the proximity of a wound of various kinds of the user, and the chemical and physical measurements in the region may have a specialized purpose relating to healing. In other examples, the bandage sensor 280 may be a useful form factor or environmentally controlled region for the inclusion of an environmental exposure sensor.

As stated above, a biometric sensor may be incorporated within a lung implant 295. A lung implant may be made into the lung epithelial tissue of a user in some examples where it may have an active or passive (sensing) role. Biometric sensors incorporated within the lung implant 295 may allow for chemical and physical monitoring. In some examples, a lung implant 295 may be used to sense biomarkers of lung cancer types. In an alternative sense, a lung implant 295 may give a means to monitor directly environmental chemical, particulate and other materials that have made their way to the lung tissue.

The biometric sensor types depicted in Fig. 2B may represent exemplary types of sensors that may be consistent with the present invention. There may be numerous other types of sensors that may be consistent with the present invention however. Furthermore, there may be examples of sensors that combine some or all the functional aspects discussed in relation to Fig. 2B which may be relevant. The present invention is not meant to be limited to those examples provided in Fig. 2B. It is important to note that the various sensors are illustrated at certain locations but may be at any location on the body depending on specific application aspects.

Non-Specific Chemical and Material Sensors

A class of sensing devices has been developed which sense the presence of chemicals in non-specific ways. There may be a number of means to accomplish this sensing. In an example, a set of shaped quartz crystals are coated with different substances such as polymers, monolayers of inorganic materials and biological antibodies as non-limiting examples. These different substances will have a unique bonding aspect to molecules in their environment. And in the case of antibodies, may be relatively specific to biological materials with certain shapes. Molecules in the environment of the sensor may adsorb onto the crystal surface and find a binding site where they may settle down. The presence of many bonded molecules on the surface layers of the crystal change the resonant frequency that the quartz crystal will oscillate at. By forcing the quartz sensors into oscillation, the resonant frequency shift of the crystal may be measured and physical principles allow for a relative calculation of the mass change of the crystal to be calculated. Different molecules may adhere to the various coatings in different yet characteristic manners. The different characteristics allow for a collection of different crystals to have the ability to distinguish different molecules, particularly when they have significantly different characteristics across the different surface types.

It is important to note that many different types of chemical sensors are known. For example, mass spectrometers and gas chromatographs may be adapted for use in accordance with the present invention.

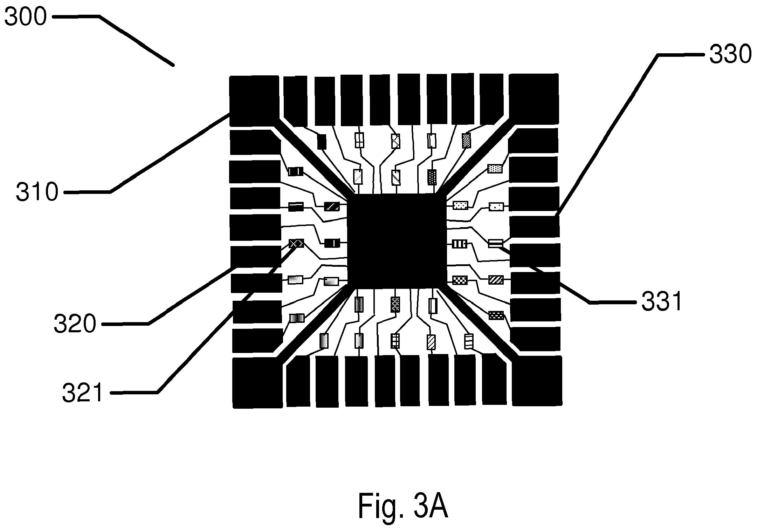

Referring to Fig. 3A, an exemplary collection of thirty or more of the crystals assembled into a "Chip" form 300 with electrical contacts to probe and measure frequency difference of the crystals for different amounts of absorbance of materials from the gas phase is illustrated. A common or ground connection 310 may connect one side of a first crystal 321 which may have a second connection 320 to apply electrical signals across the first crystal 321 to probe for its characteristics. A second crystal 331 may use the common ground connection 310 to electrically connect one side of the crystal and a second contact 330 distinct to the second crystal to simultaneously record the characteristics of the second crystal with exposure to an air sample.

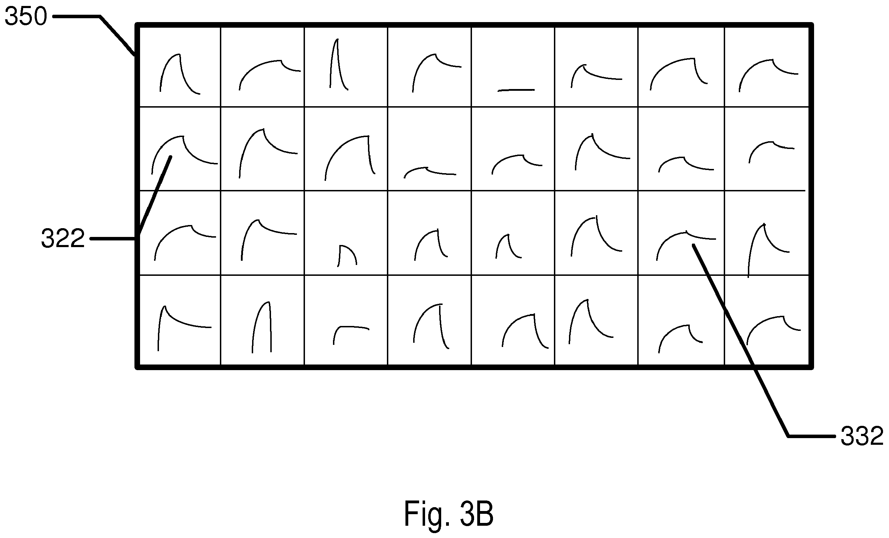

When the "chip" 300 is to be used, it may be exposed to a means to desorb any molecules from the surfaces of any of the crystals. In a non-limiting example, the crystals may be electrically heated to desorb the molecules. In some examples, the sensing device may comprise two sets of chips, where one is used to absorb and analyze gas samples and the other may be closed off from sampling so that it may be heated to clear the sensing surfaces. After the sensor cools down to an operating temperature the sensor may begin to probe the air environment and molecules may absorb onto the different crystal surfaces, and to register different absorption signals with time. Referring to Fig. 3B, a collection of exemplary responses of the thirty-two different crystals depicted in Fig. 3A may be displayed 350 showing the calculated rate of increase of mass for each of the different crystal types. The exemplary first crystal type may have a first rate of increase of mass depicted at 322 where the second crystal may have the result depicted at 332. The results in Fig. 3B may indicate that a number of different gaseous species at different concentrations may be absorbing onto the crystal array. In some examples as a detection array is utilized it may eventually be "fouled" by materials that it is exposed to, whether the intended analytes or other molecules or species in the environment. Thus, there may be cases where single use apparatus may be desirable. Therefore, analytic solutions may be implemented in a variety of ways, including as a disposable array of encapsulated sensors configured for one-time use or a disposable array that may be cleaned after one or more uses.

The crystal array may have been "trained" on known concentrations of known gas species to find the different responses for each of the different sensors that can be stored into a database. Algorithms may use these trained responses to analyze dynamic responses of the non-specific sensor to air samples and mathematically extract the highest probability gases that are present in the air. The technique may work especially well for conditions where a small number of the ambient gasses are changing at a particular time or when there are only a few gasses that are present.

In an exemplary sense, the mathematics of a combination of sensors exposed to a collection of gases may be depicted as follows. In the condition where the array of sensors behaves with linear response curves and also demonstrates additive response to analyte mixtures, then the response of a particular sensor to a mixture of $m$ analytes may be modelled as:

$$response = \sum_{k=1}^{m} concentration(k)\ sensitivity(k), \quad (1)$$

wherein concentration(k) represents the concentration("conc") of the $k^{th}$ analyte and sensitivity(k) represents the sensitivity("Sen") of the $k^{th}$ analyte on the particular sensor site. The combination of an array of $n$ sensors leads to a system of linear equations that may be represented as

$$[response(1) \dots response(n)] = [conc.(1) \dots conc.(m)]\ X \begin{bmatrix} sens(1,1) \dots sens(1,n) \\ \vdots \ddots \vdots \\ sens(m,1) \dots sens(m,n) \end{bmatrix}.$$

$$(2)$$

The resulting equations may be used to extract relationships for expected concentrations of the unknown number of analytes in the mixture. In many types of analysis, a greater number of sensors increases the accuracy of extracted results, especially when the sensing elements create significantly different responses across the gases that are present in the mixture. It may also be true that the greater the number of gases in the sample, the lower the accuracy of the responses that may be calculated.

There may be a number of different types of sensing elements that may be formulated to create non-specific sensing arrays of large numbers of sensing elements. In some examples, polymer based sensing elements may be variously doped with different compounds that tailor the surface of the polymer to have different absorbent properties to analytes. Large numbers of elements in small array systems may be formed with the processing equipment of integrated circuits for these type of polymer based sensor. In some examples, changes in dielectric constant or effective dielectric thickness of sensors that may occur when molecules bind to their surface may be sensed as changes in the capacitive nature of the probe element. For example, nanosheet-based field effect biosensors may be utilized to detect cancer marker proteins, Hereto, different binding to differently formulated surface layers which may be coated with different materials or may have "dopants" of various types implanted into the polymer sensing structures may create different changes in the capacitive nature of probe elements upon exposure.

Another type of sensing element, may be formed using carbon nanotube tips. The tips may be electrically connected to a voltage source that ramps the tips to form electric fields in the vicinity of the tips and characterize the breakdown characteristics of molecules in the gas phase. There may be other types of sensor arrays that may be formulated that provide small low cost, reusable sensing modules that may be used in the various environmental sensing uses as have been described previously. Some sensors of these types may also be able to characterize particulate matter, particularly nano-particulates present in a gas sample.

Particulate and Dust Sensors

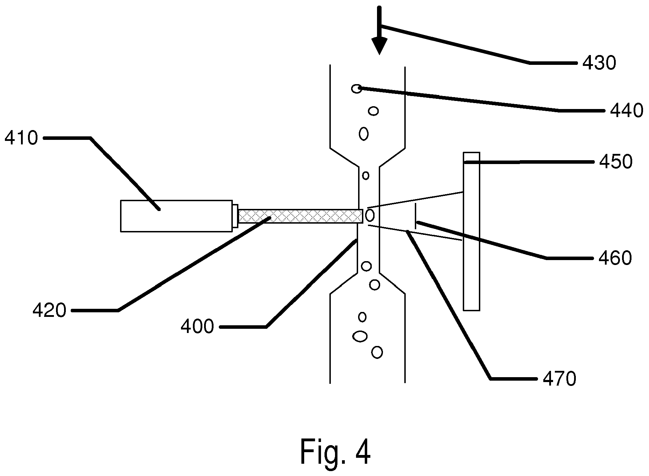

A patient with elevated or high pre-disposition to acquiring lung cancer may monitor and control exposure to particulates in his or her environment. For some of the smallest particulates such as nano-particulates, some of the non-specific chemical and material sensors may be sensitive to the binding of such nano-particulates to its surface and may be able to detect their presence. Generally other types of detectors may be used to detect levels of particulates. Referring to Fig. 4, an exemplary particulate monitoring apparatus is illustrated. A light beam is directed to a flow of air 430 in a transparent tube 400 which concentrates the air flow into a confined region. Particulates in the air flow such as particulate 440 flow into the confinement region where a laser light beam 420 from a laser light source 410 impinges on a particulate and is scattered 470 by the particulate. When there is no particulate in the beam path then the light will proceed through the transparent tube 400 and is absorbed by the beam stop 460. Thus, only light that is scattered 470 will pass by the beam stop 460 and on to a photodetector 450. In some examples, the photodetector 450 will have multiple isolated sensing regions which can discriminate an arc of scattering angles which may be used by the particulate sensor to estimate the relative size of the particulate that is scattering the light since the scattering angle may be a function of the size of the particulate.

A light scattering particle counter may be formed into a small portable sensing device that may be a wearable device. The largest energy consumption of such a device may be the incorporation of a pumping system or other air flow system which is needed to move the various particulates through the sensing region. For a wearable device, such a power source may be provided by a battery.

In some examples, a light scattering particle counter may be fixedly mounted in a particular location. The sensor may provide sensor data on an autonomous basis to databases through internet connectivity. In some examples, a particle sensor may be placed in a work place near a patient with an elevated or high pre-disposition to acquiring lung cancer. As well, a particle sensor may also be placed within a home location.

When a wearable or fixedly mounted particle counter detects an elevated level of particulate matter it can sound an audible alarm. In some examples, the sensor may communicate its sensing levels to servers through internet connections. Software algorithms may be used to process the sensor data and in some examples may be able to ascertain a location of a patient. If the patient is located in a region around a sensor during a detection of elevated particulates, a communication may be made to a device of the user such as, in a non-limiting sense, a cellular phone or smart phone. A pre-disposed patient may take protective actions based on detection of elevated particulate levels in the air such as by the wearing of protective apparel like filtering masks. In some examples, a user may leave a location or avoid going to a location when an elevated level of particulate matters is detected. By taking such actions, a pre-disposed patient may lower their cumulative exposure to environmental agents which may in turn lower the risk level for his acquiring lung cancer.

Detection levels which are correlated to a known location of the patient may also be communicated to databases which may store a record of these exposure events. The cumulative exposure values and the details of exposure events may be used by medical professionals to motivate preventive actions such as enhanced screening of patients with exposure to environmental agents.

Chemical Specific and Analytical Sensors

In some use environments, the use of chemical specific sensors or sophisticated analytical sensors may be used to detect chemicals known to be related to increased risk of obtaining lung cancer. Some chemical specific sensors may use microfluidic processing devices to perform chemical or electrochemical sensing techniques to investigate air samples for the quantitative evaluation of the gasses present including specific gasses of interest for increased risk to pre-disposed risk patients. Chemically selective field effect transistor based sensors may provide selective response to the presence of selected chemicals in gas or liquid samples. Electronic sensors based on the field-effect transistor may provide an efficient and potentially low cost sensor base that may be sensitive for various chemical and biological analyte classes. A class of field effect transistors with active channels comprising nanostructured semiconductors provides particularly sensitive and effective field effect transistor-based electronic sensors. Versions based on one-dimensional silicon nanowires and carbon nanotube and two-dimensional graphene and its derivatives are among the most promising channel materials in electronic sensors. Absorbance of gas molecules upon the channel regions of the field effect transistor create reproducible shifts in the conductivity of the channels of arrays of field effect transistors. The channels may be functionalized by various treatments of the channel region materials to chemical transformations, physical transformations and the like. A similar variation in gas sensing devices may result from functionalized metal oxide semiconductor (MOS) sensor arrays. Conductometric semiconducting metal oxide gas sensors have been well studied and characterized and constitute a desirable sensor type for the inventive concepts herein. MOS sensor arrays are desirable for gas sensing under atmospheric conditions due to the low cost and flexibility in production as well as their simplicity in use. MOS sensor arrays have been demonstrated to be able to sense large numbers of detectable gases in numerous possible application fields. In addition to the conductivity change of gas-sensing material, the detection of this reaction can be performed by measuring the change of capacitance, work function, mass, optical characteristics or reaction energy released by the gas/solid interaction. In some examples, an array of functionalized MOS sensors in which the gate is modified / treated / functionalized to have an affinity for certain molecules may operate to sense the concentration of such adsorbed gas molecules by their modulation in the work function.

In some examples, more sophisticated chemical sensing equipment may be used for environmental sensing. Techniques such as gas chromatography / mass spectrometry may provide highly accurate and sensitive sensing protocols for a wide range of analytes. There may be other techniques that provide analytical results with relatively sophisticated testing protocols such as in a non-limiting sense electrochemical sensors which may sense analytes with voltammetry analysis, inductively couple plasma atomic emission spectroscopy (ICP _AAS) or mass spectroscopy (ICP_MS) for detection of metallic constituents, and various spectroscopic technics such as gas chromatographic infrared spectroscopy (GC-IR) for characterization particularly of volatile organic carbon compounds. In some of these examples development activities for small form factor devices are ongoing, but generally these sophisticated techniques may have relatively higher cost factors and may have larger form factors for deployed sensing devices. The analytical techniques may be particularly suited for sensing of regional air quality, the results of which may be shared with databases and accessed by software algorithms to provide communication and warnings to predisposed patients that are located in the vicinity of such a system when it detects an elevated level of chemicals. In some examples, there may be situations where dedicated chemical sensors that are focused on limited or single chemical species may be used either to monitor environments or to analyze samples of breath of a patient. In some examples small infrared based detectors may be configured to detect a specific species or a combination of specific gases. The infrared signature of a specific species may absorb light at characteristic frequencies and with relative absorption rates. For certain combinations of species, there may be enough discreteness of the absorption spectra to allow for quantitative analysis of these multiple species. Diffusion controlled electrochemical based sensor are another example where an analysis system can be configured to target accurate sensing of mostly single gas systems. A combination of such specific sensors may provide a means of accurately analyzing for the presence of a specific combination of gas species.

Biomarkers/Analytical Chemistry

A biomarker, or biological marker, generally refers to a measurable indicator of some biological state or condition. The term is also occasionally used to refer to a substance the presence of which indicates the existence of a living organism. Further, life forms are known to shed unique chemicals, including DNA, into the environment as evidence of their presence in a particular location. Biomarkers are often measured and evaluated to examine normal biological processes, pathogenic processes, or pharmacologic responses to a therapeutic intervention. In their totality, these biomarkers may reveal vast amounts of information important to the prevention and treatment of disease and the maintenance of health and wellness.

Biomedical devices configured to analyze biomarkers may be utilized to quickly and accurately reveal one's normal body functioning and assess whether that person is maintaining a healthy lifestyle or whether a change may be required to avoid illness or disease. Biomedical devices may be configured to read and analyze proteins, bacteria, viruses, changes in temperature, changes in pH, metabolites, electrolytes, and other such analytes used in diagnostic medicine and analytical chemistry. As used herein, techniques to use biomedical devices to analyze biomarkers may be useful to monitor for changes in a patient's biome which may be indicative of early or advanced stages of the acquisition of lung cancer. The techniques may also probe for the presence of an infectious agent particularly of the lungs which may increase the chance of acquisition of lung cancer. As mentioned previously some, of the non-specific sensor protocols and devices may be coated with antibodies or other biological molecules that may allow for a signal change in the sensing device when a pathogen comes in contact with the device surface.

Fluorescence-Based Probe Elements for Analyte Analysis

Various types of analytes may be detected and analyzed using fluorescence based analysis techniques. A subset of these techniques may involve the direct fluorescence emission from the analyte itself. A more generic set of techniques relate to fluorescence probes that have constituents that bind to analyte molecules and in doing so alter a fluorescence signature. For example, in Förster Resonance Energy Transfer (FRET), probes are configured with a combination of two fluorophores that may be chemically attached to interacting proteins. The distance of the fluorophores from each other may affect the efficiency of a fluorescence signal emanating therefrom.

One of the fluorophores may absorb an excitation irradiation signal and may resonantly transfer the excitation to electronic states in the other fluorophore. The binding of analytes to the attached interacting proteins may disturb the geometry and cause a change in the fluorescent emission from the pair of fluorophores. Binding sites may be genetically programmed into the interacting proteins, and for example, a binding site, which is sensitive to glucose, may be programmed. In some cases, the resulting site may be less sensitive or non-sensitive to other constituents in interstitial fluids of a desired sample.

The binding of an analyte to the FRET probes may yield a fluorescence signal that is sensitive to glucose concentrations. In some exemplary embodiments, the FRET-based probes may be sensitive to as little as a 10 μM concentration of glucose and may be sensitive to up to hundreds of micromolar concentrations. Various FRET probes may be genetically designed and formed. The resulting probes may be configured into structures that may assist analysis of interstitial fluids of a subject. Alternatively, a sensing devices may present a membrane to air which allows for the diffusion of analytes through the membrane and into a fluid layer containing the FRET probes. An array of differently tuned FRET probes may provide flexible but specific sensing capable for a number of types of analytes ranging from biological molecules to environmental molecules of interest.

In some exemplary embodiments, the probes may be placed within a matrix of material that is permeable to either interstitial fluids when located in a patient, or to the fluids of the analytical device which surround the FRET probes. For example, different FRET probes may be assembled into hydrogel structures. In some exemplary embodiments, these hydrogel probes may be included into the hydrogel based processing of ophthalmic contact lenses in such a manner that they may reside in a hydrogel encapsulation that is immersed in tear fluid when worn upon the eye. In other exemplary embodiments, the probe may be inserted in the ocular tissues just above the sclera. A hydrogel matrix comprising fluorescence emitting analyte sensitive probes may be placed in various locations that are in contact with bodily fluids containing an analyte. Since the eye is exposed to the environment, it may be possible for a contact lens that is worn to provide monitoring capability for the environment of the patient. In other examples, the hydrogel with imbedded FRET probes may reside in a sensor device which contains the necessary fluorescence light sources and detectors to read out signals from the FRET probes, which may be assembled in an array format.

In an example of an ophthalmic sensor within the body of a pre-disposed patient, the fluorescence probes may be in contact with interstitial fluid of the ocular region near the sclera. In these cases, where the probes are invasively embedded, a sensing device may provide a radiation signal incident upon the fluorescence probe from a location external to the eye such as from an ophthalmic lens or a handheld device held in proximity to the eye. FRET probes may be engineered to be sensitive to proteins and hormones that may be indicative of the growth of lung cancer as has been previously described.

In other exemplary embodiments, the probe may be embedded within an ophthalmic lens in proximity to a fluorescence-sensing device that is also embedded within the ophthalmic lens. In some exemplary embodiments, a hydrogel skirt may encapsulate both an ophthalmic insert with a fluorescence detector as well as a FRET-based analyte probe. Further enablement for the use of fluorescence detectors in biomedical devices may be found as set forth in United States Patent Application 14/011,902 filed August 28, 2013, which is incorporated herein by reference.

In some examples, a non-specific chemical detector may be fashioned based on fluorescence based analysis. The analysis device may be configured to interact with gaseous chemicals in the breath of patients. When breathed upon, a sensor plate which may include dozens of different sensor "spots" may interact with the various gases in the patient's breath. The test apparatus may then have its fluorescence spectrum characterized. Much like other non-characteristic sensors, the probe may be "trained" to obtain the unique spectral response for different analytes and then these characteristic results may be assembled into a database that is used in analyzing the overlap result.

In some examples, the absolute levels of analytes may form only one basis for determining a concern. Alternatively, or in addition to comparing measurements to a predetermined or absolute threshold, a baseline for the healthy patient may be established and the detectors may be utilized to detect changes from this baseline. A change in measurements over time may signal the approach of a condition that will inevitably exceed the threshold, and detection of the time dependent change may afford earlier detection effectiveness. It is also possible that a spectrum of disease states and concomitant analyte generation may vary depending on the exact state of disease. In such cases, it may not be possible to determine a completely effective threshold action value, thus the use of changes from a baseline value for a given individual may be an effective method of detection and proactive treatment.

Various types of non-specific sensors may be configured into an array which may be used to sense ambient for chemical composition. A clearly important type of ambient that may be sensed from a health perspective may be the breath of a patient. The breath of a patient may contain a large array of different chemical constituents including in non-limiting examples 2-ethylhexanol, 3-methylhexane, 5-ethyl-3-methyloctane, acetone, ethanol, ethylacetate, ethylbenzene, isononane, isoprene, nonanal, styrene, toluene, and undecane. For various diseases the relative levels of these chemicals may differ significantly. Detection of these constituents and an ability to monitor a portfolio of the chemicals in a sample of a patients breath may be understanding of disease states that may occur in patients. For example, studies have shown that in the breath of patients with lung cancer that the breath of these patients contains significant levels of some of these chemicals. In examples the chemicals typically present may include: 2-ethylhexanol; 3-methylhexane; ethanol; isononane; isoprene; nonanal; styrene; and toluene. Other important analytes may be present in lower levels that these examples.

It has been demonstrated that a cancer-related gas detector may be assembled with a relatively small (about 50 millimeter diameter) circular plate. On the plate are sensitive spots to form a fluorescent cross-responsive sensor array. The sensor may consist of a disposable array with dozens of chemically responsive spots located around the sensor edge. The spots may then be filled with different samples of sensitive synthetic compounds. Interaction of such a fluorescence cross-responsive array with specific analytes or volatile organic compound gases may cause the characteristic shifts in fluorescence characteristics of the spots. By collecting the fluorescent emission spectrum of the sensor array before and after the reaction, a dataset may be obtained which may be analyzed across a database of known analytes to determine the best fit set of chemicals that can explain the shifts in the fluorescence response of the dozens of test sites. Probes based on these principles may be fashioned into various form factors which may interact with the breath of a patient.

Sensing Devices with Event Coloration Mechanism

Another method of detecting analytes may be a passive coloration scheme wherein analytes may strictly bind to a reactive compound resulting in a color change which may indicate the presence of a specific analyte. Such probes may be formulated into stand-alone sensing elements which use spectroscopy to detect changes in the coloration scheme. In other examples, the coloration may be detected as a signal that a pre-disposed risk lung cancer patient may visually see. In the following paragraphs description of such a visual scheme in a contact lens where the optic zone of the lens may change color at least in a part is described. It may be noted that the use of the various coloration technology may alternatively be disposed into portable sensors with or without spectroscopic readout of various kinds that are not worn on the eye.

In proceeding with an ophthalmic example, an event coloration mechanism may comprise a reactive mixture, which, for example, may be added to, printed on, or embedded in a rigid insert of an ophthalmic device, such as through thermoforming techniques. Alternatively, the event coloration mechanism may not require a rigid insert but instead may be located on or within a hydrogel portion, for example, through use of printing or injection techniques.

The event coloration mechanism may comprise a portion of a rigid insert that is reactive to some component of the transient tear fluid or some component within an ophthalmic lens. For example, the event may be a specific accumulation of some precipitant, such as, lipids or proteins, on either or both the rigid ophthalmic insert and a hydrogel portion, depending on the composition of the ophthalmic lens. The accumulation level may "activate" the event coloration mechanism without requiring a power source. The activation may be gradual wherein the color becomes more visible as the accumulation level increases, which may indicate when the ophthalmic lens needs to be cleaned or replaced.

Alternatively, the color may only be apparent at a specific level. In some embodiments, the activation may be reversible, for example, where the wearer effectively removes the precipitant from the hydrogel portion or the rigid insert. The event coloration mechanism may be located outside the optic zone, which may allow for an annular embodiment of the rigid insert. In other embodiments, particularly where the event may prompt a wearer to take immediate action, the event coloration mechanism may be located within the optic zone, allowing the wearer to see the activation of the event coloration mechanism.

In some other embodiments, the event coloration mechanism, may comprise a reservoir containing a colored substance, for example, a dye. Prior to the occurrence of the event, the reservoir may not be visible. The reservoir may be encapsulated with a degradable material, which may be irreversibly degraded by some constituent of the tear fluid, including, for example, proteins or lipids. Once degraded, the colored substance may be released into the ophthalmic lens or into a second reservoir. Such an embodiment may indicate when a disposable ophthalmic lens should be disposed of, for example, based on a manufacturer's recommended parameters.

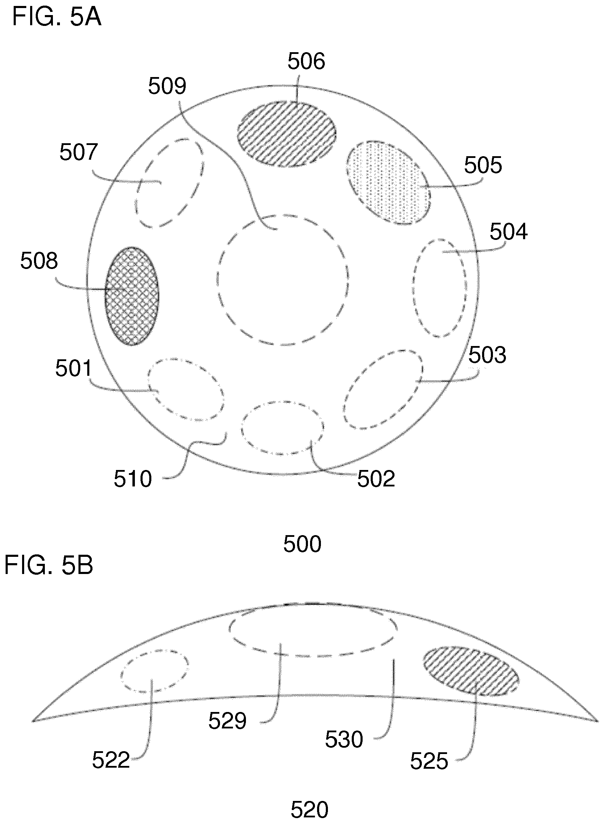

Proceeding to Figs. 5A and 5B, an exemplary embodiment of an ophthalmic lens 500 with multiple event coloration mechanisms 501-508 is illustrated. In some embodiments, the event coloration mechanisms 501-508 may be located within the soft, hydrogel portion 510 of the ophthalmic lens 500 and outside the optic zone 509.

Such embodiments may not require a rigid insert or media insert for functioning of the event coloration mechanisms 501-508, though inserts may still be incorporated in the ophthalmic lens 500 allowing for additional functionalities. In some embodiments, each event coloration mechanism 501-508 may be separately encapsulated within the soft, hydrogel portion 510 of the ophthalmic lens 500. The contents of the event coloration mechanisms 501-508 may include a compound reactive to some condition, such as temperature, or component of tear fluid, such as a biomarker.

In some embodiments, each event coloration mechanism 501-508 may "activate" based on different events. For example, one event coloration mechanism 508 may comprise liquid crystal that may react to changes in temperatures of the ocular environment, wherein the event is a fever. Other event coloration mechanisms 502-506 within the same ophthalmic lens 500 may react to specific pathogens, for example, those that may cause ocular infections or may be indicative of non-ocular infections or diseases, such as keratitis, conjunctivitis, corneal ulcers, and cellulitis. Such pathogens may include, for example, *Mycobacterium tuberculosis, Acanthamoeba keratitis, Pseudomoha aerugihosa, Neisseria gohorrhoeae*, and *Staphylococcus* and *Streptococcus* strains, such as *S. aureus*. Some of these pathogens may be risk factors for patients with elevated or high pre-disposition to acquisition of lung cancer, others may be opportunistic infections which may occur in the presence of a lung cancer state before or after it is being treated.

The event coloration mechanisms 501-507 may be encapsulated with a compound that may be selectively permeable to a component of tear fluid. In some embodiments, the event coloration mechanisms 502-506 may function by agglutination, such as through a coagulase test, wherein a higher concentration of the pathogen may adhere to a compound within the event coloration mechanisms 502-506 and may cause clumping or the formation of precipitate. The precipitate may provide coloration or may react with another compound in the event coloration mechanisms 502-506 through a separate reaction. Alternatively, the event coloration mechanisms 502-506 may comprise a reagent that colors upon reaction, such as with some oxidase tests.

As shown in cross section 520 in Fig. 5B, the event coloration mechanisms 522, 525 may be located in the periphery of the ophthalmic lens without altering the optical surface of the hydrogel portion 530. In some embodiments, not shown, the event coloration mechanisms may be located at least partially within the optic zone 529, alerting the wearer of the event. The locations of the event coloration mechanisms 522, 525 may be varied within a single ophthalmic lens 500, with some in the periphery and some within the optic zone 529.

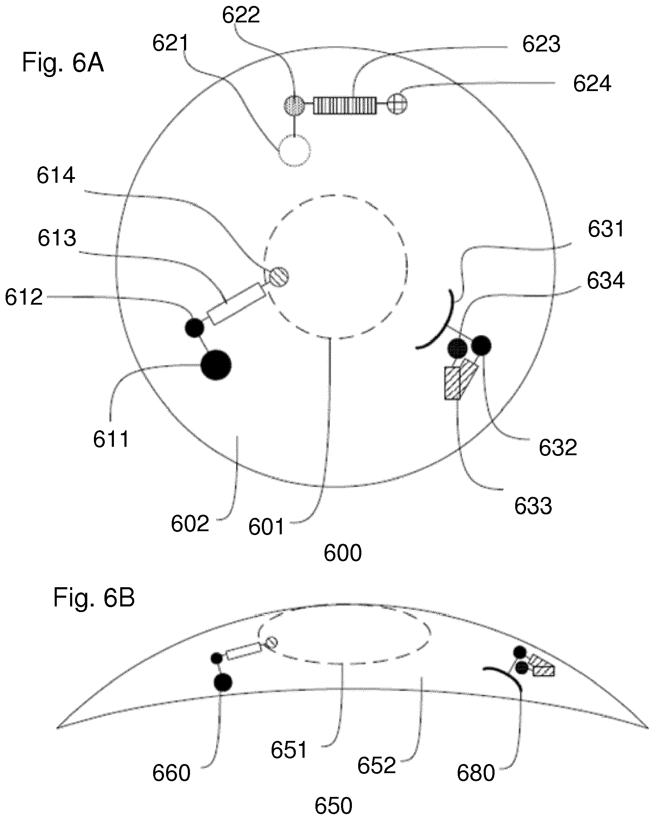

Referring to Figs. 6A and 6B, an alternative exemplary embodiment of an ophthalmic lens 600 with an optic zone 601 and a non-optic zone 602 with event coloration mechanisms 611-614, 621-624, and 631-634 is illustrated. In some such embodiments, the event mechanisms 611-614, 621-624, and 631-634 may include a reactive molecule 612-614, 622-624, and 632-634 respectively, anchored within the ophthalmic lens 600. The reactive molecule 612-614, 632-634 may comprise a central binding portion 613, 633 flanked by a quencher 612, 632 and a coloration portion 614, 634, for example, a chromophore or fluorophore. Depending on the molecular structure, when a specified compound binds to the binding portion 613, 633, the coloration portion 614, 634 may shift closer to the quencher 612, reducing coloration, or may shift away from the quencher 632, which would increase coloration. In other embodiments, the reactive molecule 622-624 may comprise a binding portion 623 flanked by Förster resonance energy transfer pairs 622, 624. FRET pairs 622, 624 may function similarly to a quencher 612, 632 and chromophore (the coloration portion) 614, 634, though FRET pairs 622, 624 may both exhibit coloration and, when in close proximity to each other, their spectral overlap may cause a change in coloration.

The reactive molecule 612-614, 622-624, and 632-634 may be selected to target specific compounds within the tear fluid. In some embodiments, the specific compound may directly indicate the event. For example, where a level of glucose in the tear fluid is the event, the reactive molecule 612-614, 622-624, and 632-634 may directly bind with the glucose. Where the event is the presence or concentration of a pathogen, for example, a particular aspect of that pathogen may bind with the reactive molecule 612-614, 622-624, and 632-634. This may include a unique lipid or protein component of that pathogen. Alternatively, the specific compound may be an indirect indicator of the event. The specific compound may be a byproduct of the pathogen, such as a particular antibody that responds to that pathogen.

As illustrated in cross section in Fig. 6B, the placement of the reactive molecules 660, 680 within the ophthalmic lens 650 may be varied within the hydrogel 652. For example, some reactive molecules 680 may be entirely in the periphery with no overlap with the optic zone 651. Other reactive molecules 660 may at least partially extend into the optic zone 651. In some such embodiments, the reactive molecules 660 may extend into the optic zone 651 in some configurations of that reactive molecule 660, such as when the event has occurred, which may alert the wearer of the event.

Further enablement for the use of event detectors in biomedical devices may be found as set forth in United States Patent Application 13/899,528 filed May 21, 2013, which is incorporated herein by reference.

Quantum-Dot Spectroscopy

Small spectroscopy devices may be of significant aid in creating biomedical devices with the capability of measuring and controlling concentrations of various analytes for a user. Current micro spectrometer designs mostly use interference filters and interferometric optics to measure spectral responses of mixtures that contain materials that absorb light. In some examples a spectrometer may be formed by creating an array composed of quantum-dots. A spectrometer based on quantum-dot arrays may measure a light spectrum based on the wavelength multiplexing principle. The wavelength multiplexing principle may be accomplished when multiple spectral bands are encoded and detected simultaneously with one filter element and one detector element, respectively. The array format may allow the process to be efficiently repeated many times using different filters with different encoding so that sufficient information is obtained to enable computational reconstruction of the target spectrum. An example may be illustrated by considering an array of light detectors such as that found in a charge-coupled device (CCD) camera. The array of light sensitive devices may be useful to quantify the amount of light reaching each particular detector element in the CCD array. In a broadband spectrometer, a plurality, sometimes hundreds, of quantum-dot based filter elements are deployed such that each filter allows light to pass from certain spectral regions to one or a few CCD elements. An array of hundreds of such filters laid out such that an illumination light passed through a sample may proceed through the array of Quantum Dot (referred to as QD) Filters and on to a respective set of CCD elements for the QD filters. The simultaneous collection of spectrally encoded data may allow for a rapid analysis of a sample.

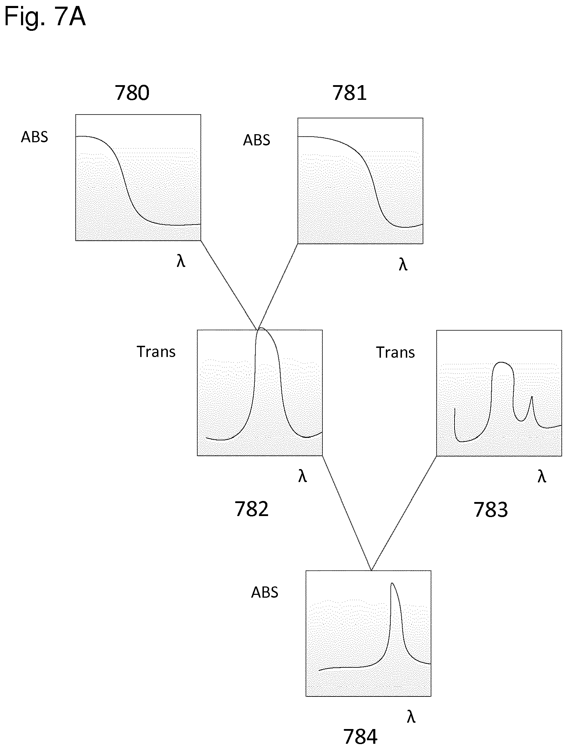

Narrow band spectral analysis examples may be formed by using a smaller number of QD filters surrounding a narrow band. In Fig. 7A an illustration of how a spectral band may be observed by a combination of two filters is illustrated. It may also be clear that the array of hundreds of filters may be envisioned as a similar concept to that in Fig. 7A repeated many times.

In Fig. 7A, a first QD filter 780 may have an associated spectral absorption response as illustrated and indicated as ABS on the y-axis. A second QD filter 781 may have a shifted spectral absorption associated with a different nature of the quantum-dots included in the filter, for example, the QDs may have a larger diameter in the QD filter 781. The difference curve of a flat irradiance of light of all wavelengths (white light) may result from the difference of the absorption result from light that traverses filter 781 and that traverses filter 780. Thus, the effect of irradiating through these two filters is that the difference curve would indicate spectral response in the depicted transmission band 782, where the y-axis is labelled Trans to indicate the response curve relates to transmission characteristics. When an analyte is introduced into the light path of the spectrometer, where the analyte has an absorption band in the UV/Visible spectrum, and possibly in the infrared, the result would be to modify the transmission of light in that spectral band as shown by spectrum 783. The difference from 782 to 783 results in an absorption spectrum 784 for the analyte in the region defined by the two quantum-dot filters. Therefore, a narrow spectral response may be obtained by a small number of filters. In some examples, redundant coverage by different filter types of the same spectral region may be employed to improve the signal to noise characteristics of the spectral result.

The absorption filters based on QDs may include QDs that have quenching molecules on their surfaces. These molecules may stop the QD from emitting light after it absorbs energy in appropriate frequency ranges. More generally, the QD filters may be formed from nanocrystals with radii smaller than the bulk excitation Bohr radius, which leads to quantum confinement of electronic charges. The size of the crystal is related to the constrained energy states of the nanocrystal and generally decreasing the crystal size has the effect of a stronger confinement. This stronger confinement affects the electronic states in the quantum-dot and results in an increase in the effective bandgap, which results in shifting to the blue wavelengths both of both optical absorption and fluorescent emission. There have been many spectral limited sources defined for a wide array of quantum-dots that may be available for purchase or fabrication and may be incorporated into biomedical devices to act as filters. By deploying slightly modified QDs such as by changing the QD's size, shape and composition it may be possible to tune absorption spectra continuously and finely over wavelengths ranging from deep ultraviolet to mid-infrared. QDs may also be printed into very fine patterns.

Biomedical Devices with Quantum-Dot Spectrometers

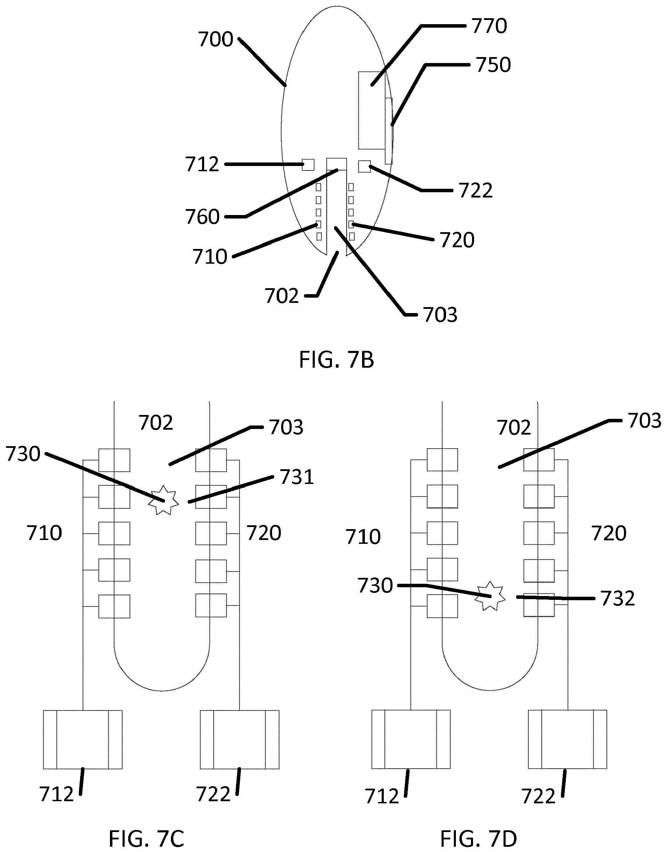

Fig. 7B illustrates an exemplary QD spectrometer system in a biomedical device 700. The illustration in Fig. 7B may utilize a passive approach to collecting samples wherein a sample fluid passively enters a channel 702. The channel 702 may be internal to the biomedical device 700 in some examples and in other examples, as illustrated, the biomedical device 700 may surround an external region with a reentrant cavity. In some examples where the biomedical device 700 creates a channel of fluid external to itself, the device 700 may also contain a pore 760 to emit reagents or dyes to interact with the external fluid in the channel region. Such biomedical devices may be included in various types of sensing locations including in a non-limiting sense, dental appliances and implants, eating implements and the like. Specific probing devices, which may require an overt testing operation by the user, such as a probe in the shape of a tongue depressor may also function by being placed into the mouth of a patient who is predisposed to the acquisition of lung cancer and may sense the spectral characteristics of the patient's breath.

In a non-limiting sense, the passive sampling may be understood with reference to an example where the biomedical device 700 may be a swallowable pill. The pill may comprise regions that emit medicament 750 as well as regions that analyze surrounding fluid such as gastric fluid for the presence of an analyte, where the analyte may be the medicament, for example, but also may be evidence of environmental agents that the patient has been exposed to that have made their way down the esophagus and into the gastric fluid. The pill may contain controller 770 regions. An analysis region 703 may comprise a reentrant channel 702 within the biomedical pill device that allows external fluid to passively flow in and out of the channel. When an analyte, for example, in gastric fluid, diffuses or flows into the channel 702 it becomes located between the analysis regions 703 as depicted in Fig. 7B.

Referring now to Fig. 7C, once an analyte diffuses or otherwise enters the quantum-dot spectrometer channel which shall be referred to as the channel 702, a sample 730 may pass in the emission portion of a quantum-dot (QD) emitter 710. The QD emitters 710 may receive information from a QD emitter controller 712 instructing the QD emitters 710 to emit an output spectrum of light across the channel 702.

In some examples, the QD emitter 710 may act based on emission properties of the quantum-dots. In other examples, the QD emitter may act based on the absorption properties of the quantum-dots. In the examples utilizing the emission properties of the quantum-dots, these emissions may be photostimulated or electrically stimulated. In some examples of photostimulation; energetic light in the violet to ultraviolet may be emitted by a light source and absorbed in the quantum-dots. The excitation in the QD may relax by emitting photons of characteristic energies in a narrow band. As mentioned previously, the QDs may be engineered for the emission to occur at selected frequencies of interest.

In a similar set of examples, QDs may be formed into a set of layers. The layers may place the QDs between electrically active layers that may donate electrons and holes into the QDs. These excitations, due to the donations of electrons and holes may similarly stimulate the QDs to emit characteristic photons of selected frequency. The QD emitter 710 may be formed by inclusion of nanoscopic crystals, that function as the quantum-dots, where the crystals may be controlled in their growth and material that are used to form them before they are included upon the emitter element.

In an alternative set of examples, where the QDs act in an absorption mode a combination of a set of filters may be used to determine a spectral response in a region. This mechanism is described in a prior section in reference to Fig. 7A. Combinations of QD absorption elements may be used in analysis to select regions of the spectrum for analysis.

In either of these types of emission examples, a spectrum of light frequencies may be emitted by QD emitter 710 and may pass thru the sample 730. The sample 730 may absorb light from some of the emitted frequencies if a chemical constituent within the sample is capable of absorbing these frequencies. The remaining frequencies that are not absorbed may continue on to the detector element, where QD receivers 720 may absorb the photons and convert them to electrical signals. These electrical signals may be converted to digital information by a QD detector sensor 722. In some examples the sensor 722 may be connected to each of the QD receivers 720, or in other examples the electrical signals may be routed to centralized electrical circuits for the sensing. The digital data may be used in analyzing the sample 730 based on pre-determined values for QD wavelength absorbance values.

In Fig. 7D, the QD system is depicted in a manner where the sample is passed in front of spectral analysis elements that are spatially located. Figs. 7C and 7D depict such movement as the difference between the locations of the sample 730 which has moved from a first location 731 along the analysis region to the new location 732. In other examples the QDs may be consolidated to act in a single multidot location where the excitation means and the sensing means are consolidated into single elements for each function. Some biomedical devices may have space limitations for a spectrometer comprising more than a hundred quantum-dot devices, but other biomedical devices may have hundreds of quantum-dot devices which allow for a full spectrographic characterization of analyte containing mixtures.

The QD analytical system may also function with microfluidic devices to react samples containing analytes with reagents containing dyes. The dye molecules may react with specific analytes. As mentioned previously, an example of such a binding may be the FRET indicators. The dye molecules may have absorption bands in the ultraviolet and visible spectrum that are significantly strong, which may also be referred to as having high extinction coefficients. Therefore, small amounts of a particular analyte may be selectively bound to molecules that absorb significantly at a spectral frequency, which may be focused on by the QD analytical system. The enhanced signal of the dye complex may allow for more precise quantification of analyte concentration.

Although described in concert with a swallowable pill it may be obvious how a quantum dot spectroscopy device may represent a small and flexible spectroscopy apparatus that may be incorporated into various sensing devices as have been described in the specification herein.

Exemplary Monitoring Paradigms

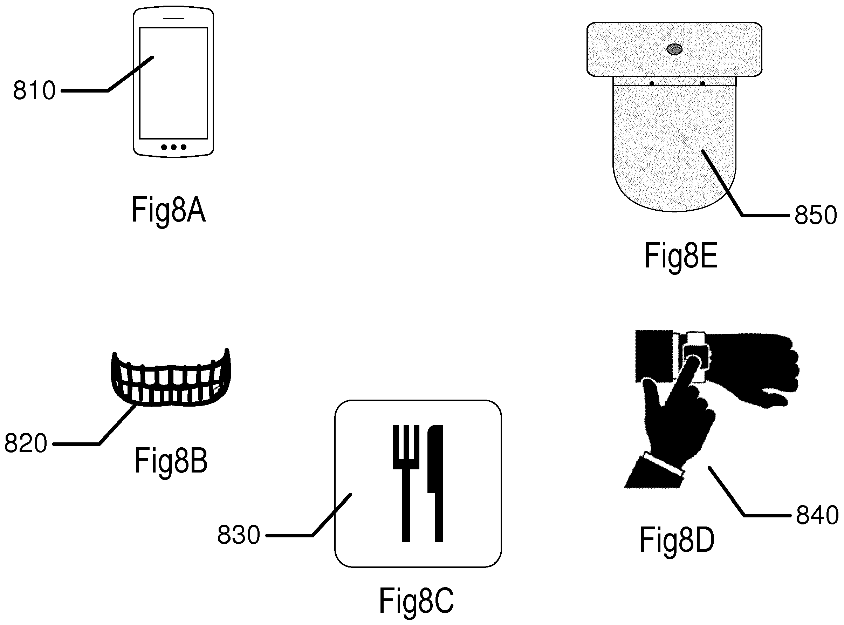

Referring now to Figs. 8A-8E, a number of exemplary means of deploying a monitoring apparatus are illustrated. Some of the illustrated means have been discussed in previous sections, but are brought here to illustrate the type of characteristics of personal monitoring that may have relevance to the monitoring of a patient who has an elevated or high propensity of acquiring lung cancer. The lungs may be distinct because they have such a significant surface area that is in contact with external air for an internal organ. The risks of environmental exposures in general may relate to the exposure of an agent in the environment to an individual where the agent is inhaled into the lung tissue of the predisposed patient. Thus, it may be advantageous to define sensing modalities that interact in easy ways with the potential exposure of the patient. It is important to understand that routine household chores, for example cleaning, as well as more involved work around the house, for example, carpentry and dry walling, may place an individual with a genetic predisposition at risk. Household cleaners, dust, drywall compound and sawdust are amongst the materials that may potentially cause a problem.

For example, a first modality may be to associate a sensing device with an electronic means that may have computational abilities, may have communication capabilities and desirably may interact with the air proximate to the mouth of a patient. A cell phone 810 in Fig. 8A may provide an excellent example of such an apparatus. In normal operation, a cell phone is frequently held to a user's mouth as he or she speaks into the phone. Sensing equipment may therefore sense the breath of the user. In some examples, the sensing equipment may monitor the air in its vicinity and therefore due to its frequent proximity to a user's mouth it will frequently monitor the air in extremely relevant manners. The cell phone 810 may include various sensing devices that may be part of the phone, or may be connected to the phone as a case or an attachment. The cell phone 810 also is an ideal means to deliver various communication to a user. Through cellular, wireless, Bluetooth or other communication means if enabled, the cell phone can receive messages and communicate them via video screens, vibrational transducers, and audio speakers as non-limiting examples.

A second exemplary modality may be illustrated in Fig. 8B as a dental appliance 820. The dental appliance may be a brace or bridge that may be placed into the mouth or reside in the mouth. In other examples, an oral appliance may include various sensing devices that may sense the breath of a user, the saliva of a user, or both. As has been discussed each may contain chemicals and particles that are in the environment. In some examples, chemicals and gasses may be released by the user's body which may indicate a status related to lung cancer.

A third exemplary modality may be illustrated in Fig. 8C as eating appliances 830. In some examples, implements that are used for eating may include sensors to sense the breath of an individual who is using them. In some examples, the saliva of the user may also be sensed by the eating appliances 830 such as a fork, knife and/or spoon.

A fourth exemplary modality for personal monitoring may be illustrated in Fig 8D as a wrist based sensor 840 such as a smart watch or an electronic bracelet. The wrist based sensor 840 may include a device that may monitor the air in its vicinity. The wrist based sensor 840 may include various sensing devices that may be part of a smart watch, or may be connected to the watch as a case or an attachment. In some examples, the wrist based sensor 840 may be a bracelet that includes sensing devices. The wrist based sensor 840 also may be an ideal means to deliver various communication to a user. Through cellular, wireless, Bluetooth or other communication means if enabled, the smart watch can receive messages and communicate them via video screens, vibrational transducers, and audio speakers as non-limiting examples.

A fifth exemplary modality for personal sensing may be illustrated in Fig. 8E as a sensor-enabled sanitary device 850 such as a toilet equipped with sensors. In excrement from a user there may be numerous chemicals that may indicate a disease state such as lung cancer. A sanitary device may be able to facilitate the acquisition of samples that may be analyzed. In some examples, a personal device of the user such as a smart phone or a smart watch may communicate with the sensor enabled sanitary device 850 and communicate information identifying the user with any sample obtained. Other sanitary sensors may include sanitary napkins, tampons and adult diapers.

There may be many other types of personal sensing modalities that may allow for the sensing of either user proximate environmental conditions or alternatively user biomedical status. The examples discussed are not meant to limit the diversity of different manners and methods under which sensing may occur. The results of the sensing may provide important information for users with pre-disposition to the acquisition of lung cancer.

Methods

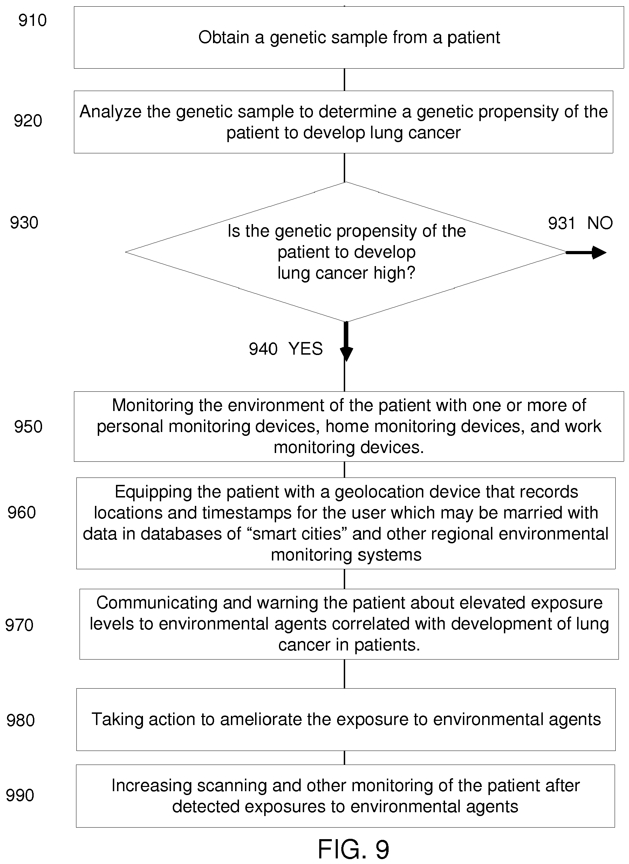

There are numerous methods by which specialized sensor technology may be deployed to assist patients with elevated or high pre-disposition to the acquisition of lung cancer in following practices which reduce the chance the individual has for acquiring lung cancer by lowering his or her exposure to environmental agents. Referring to Fig. 9, a first example of such methodology is illustrated. At 910, a patient may have a sample of their genetic material taken and submitted for analysis. Including the examples set forth above as well as those identified in the future, there may be numerous genetic locations where variations may be analyzed and used to determine a genetic propensity of the patient to develop lung cancer as shown in step 920. In some examples, it may be determined at step 930 that the patient does not have an elevated or high propensity to develop lung cancer as is depicted at step 931. In other examples, the patient may have an elevated or high propensity to develop lung cancer as illustrated at step 940. For these patients, a prudent step illustrated at step 950 may include monitoring of the environment of the patient with one or more of personal monitoring devices, home monitoring devices and work monitoring devices as the many examples discussed herein has depicted. In some examples, as illustrated in the optional step 960, the patient who is predisposed may be equipped with a geolocation device which may determine his or her location along with accurate time and date stamps in a database. The location and time data may be married with other databases such as databases of "Smart Cities" where cities deploy a myriad of sensors in their neighborhoods and pass that information into large city related databases. Environmental sensing is an important type of data that is being sensed in current operations. There may be numerous agencies and organizations that sense the environment of specific locations and then store the analysis results. Under free or fee-based models the data may be accessed by applications that the lung patient may have access to. Based on the systems sensors or on the marriage of location and time state data with environmental databases, a state of elevated exposure may be detected by algorithms running in applications of the user. At step 970, the patient may receive a warning that is communicated to him or her by the algorithm. The warning may indicate to the user that an environmental agent has been detected in an abundant level and may suggest actions that the user may take. At step 980, the user may take one or more of the actions to ameliorate the exposure to the environmental agents. In some examples, the patient may exit the area of the sensed elevated level of environmental agents. In some other examples, the patient may use protective apparel to shield him or herself from exposure, for example, a simple surgical mask. At step 990, because the patient may have been exposed to an elevated level of an environmental agent which may be suspected to increase the potential of the patient to acquiring lung cancer, he or she may be identified as a patient where increased medical scanning and monitoring for deleterious effects of the historic exposures of the patient to the environmental agents is performed proactively. In addition to monitoring, the patient may be given some form of treatment to treat, reverse the damage if possible, or protect the patient from potential future harm. Even with ameliorative treatment, the measurements of exposure may still guide for an enhanced monitoring scheme for development of cancer which may include medical imaging studies and genetic monitoring for mutation events, as well as standard monitoring for chemical species in the breath of the patient at frequent intervals.

In some examples, it may be possible to stratify the risk potential of a patient with some level of genetic variation related to the development of lung cancer into more levels than just high when there is evidence of variation and low when there is not. There may be some "grey" levels which may be indicated by the presence of certain types of variation or based on the number of sites which show variation as examples. There may be some different methodology for patients with elevated but not high levels of risk potential.

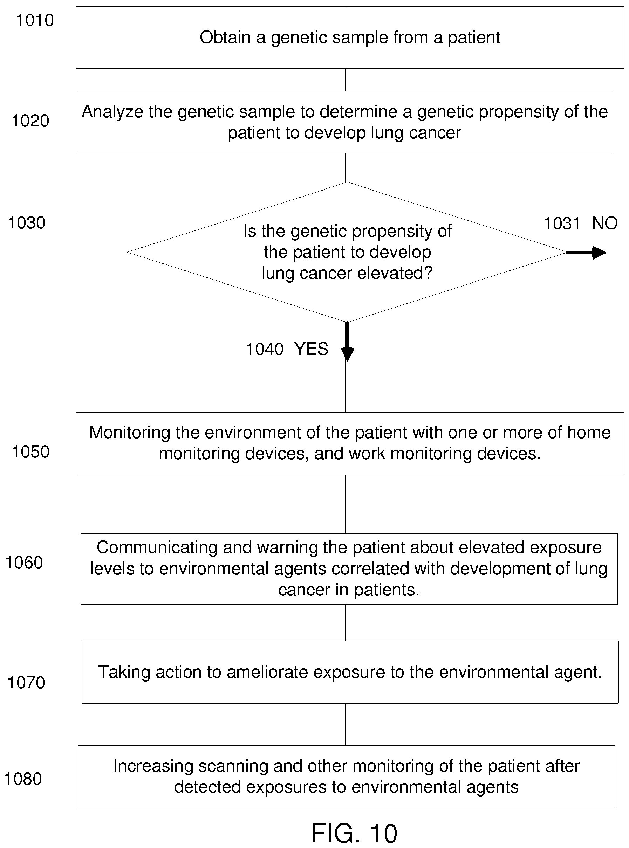

Referring to Fig. 10, an example of different methodology related to patients at genetically elevated risk of developing lung cancer where the risk is stratified into an intermediate risk level. In the example of Fig. 10, when compared to that of Fig. 9, the level of monitoring may be modified as a non-limiting example. Whereas a high risk patient may have monitors associated with work, home and personal space as well as geolocation based tracking and risk interpolation, an elevated risk patient may only have his workspace and home monitored as non-limiting examples.

Referring to Fig. 10, at 1010, a patient may have a sample of their genetic material taken and submitted for analysis. Given the rapidly developing and evolving understanding of the genetic correlation with lung cancer risk, the monitoring/alert devices in accordance with the present invention may comprise update mechanisms such that any new screening criteria and/or processes may be directly uploaded or otherwise made available to the monitoring/alert devices of the present invention. In some examples, portions of the hardware of monitoring/alert devices may be updated in a similar manner as the software updates or in addition to the software updates to bring the monitoring/alert devices in line with newly developed understanding.

Including the examples set forth above as well as those identified in the future, there may be numerous genetic locations where variations may be analyzed and used to determine a genetic propensity of the patient to develop lung cancer as shown in step 1020. In some examples, it may be determined at step 1030 that the patient does not have even an elevated propensity to develop lung cancer as is depicted at step 1031. In other examples, the patient may have just an elevated propensity to develop lung cancer as illustrated at step 1040. For these patients, a prudent step illustrated at step 1050 may include monitoring of the environment of the patient with one or more of home monitoring devices and work monitoring devices as the many examples discussed herein has described. It may be determined that the generalized sensing of the external environment is sufficient with home and work monitoring devices and therefore that correlation to environmental databases is not necessary.

At step 1060, the patient may receive a warning that is communicated to him or her by the algorithm. The warning may indicate to the user that an environmental agent has been detected in an abundant level and may suggest actions that the user may take. At step 1070, the user may take one or more of the actions to ameliorate the exposure to the environmental agents. In some examples, the patient may exit the area of the sensed elevated level of environmental agents. In some other examples, the patient may use protective apparel to shield him or herself from exposure. At step 1080, because the patient may have been exposed to an elevated level of an environmental agent which may be suspected to increase the potential of the patient to acquiring lung cancer, he or she may be identified as a patient where increased medical scanning and monitoring for deleterious effects of the historic exposures of the patient to the environmental agents is performed proactively.

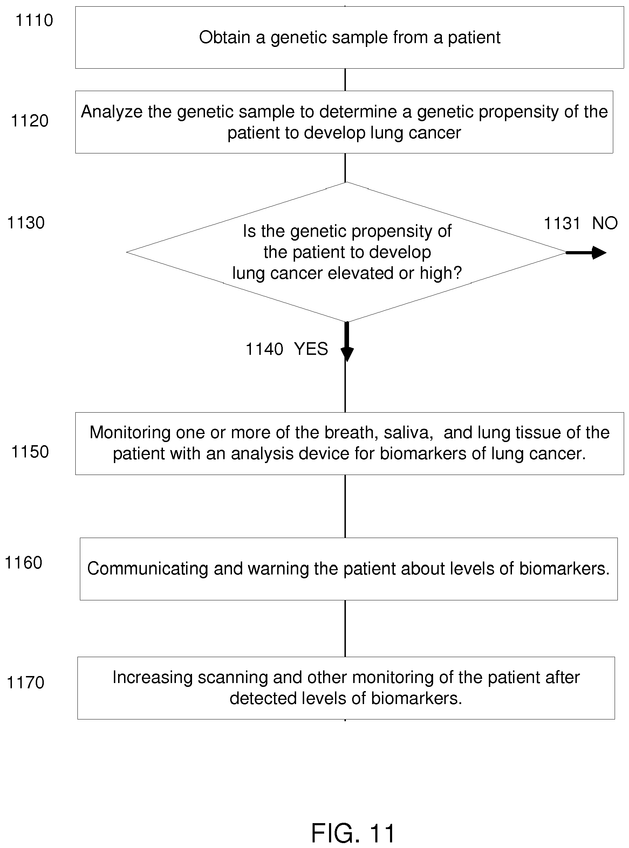

There may also be methods with use genetic assessment to drive monitoring of an individual for the presence of biomarkers that may indicate early stages or general occurrence of lung cancer. Referring to Fig. 11, an example of methodology related to detecting genetic propensity and then routinely monitoring high or elevated risk patients for the presence of analytes indicating advancement of disease. Referring to Fig. 11, at 1110, a patient may have a sample of their genetic material taken and submitted for analysis. Including the examples set forth above as well as those identified in the future, there may be numerous genetic locations where variations may be analyzed and used to determine a genetic propensity of the patient to develop lung cancer as shown in step 1120. In some examples, it may be determined at step 1130 that the patient does not have a high or elevated propensity to develop lung cancer as is depicted at step 1131. In other examples, the patient may have a high or elevated propensity to develop lung cancer as illustrated at step 1140. For these patients, a prudent step illustrated at step 1150 may include monitoring one or more of the breath, saliva and lung tissue of the patient with one or more types of monitoring devices for the analysis of the presence of biomarkers of lung cancer.

At step 1160, in some cases sensors may detect potential elevated levels of biomarkers and the patient may receive a warning that is communicated to him or her by a monitoring systems which interfaces the various sensors. The warning may indicate to the user that biomarker may have been detected in an elevated level and may suggest actions that the user may take. At step 1170, because the patient may have indication via certain biomarkers that may indicate that the high or elevated propensity patient's tissues are undergoing changes consistent with lung cancer, he or she may be identified as a patient where increased medical scanning and monitoring for deleterious effects of the historic exposures of the patient to the environmental agents is performed proactively.

Although shown and described is what is believed to be the most practical and preferred embodiments, it is apparent that departures from specific designs and methods described and shown will suggest themselves to those skilled in the art and may be used without departing from the spirit and scope of the invention. The present invention is not restricted to the particular constructions described and illustrated, but should be constructed to cohere with all modifications that may fall within the scope of the appended claims.

Further examples of the present disclosure are provided in the following numbered list.

1. A system for detecting a form of cancer, the system comprising:

a means for obtaining genetic data of a patient to determine variation at target locations of the patient's genome, wherein the variations are related to an increased risk of having the form of cancer;

a first database record, wherein the first database record contains records of familial health history;
a second database record, wherein the second database record contains one or more of a null dataset and a record of an environmental exposure of the patient;
a first sensor system, wherein the first sensor system is configured to sense biomarkers related to a cancer state, wherein the biomarkers emanate from the user;
a second sensor system, wherein the second sensor system is configured to sense the local environment of the user for one or more of particulate matter and chemical pollutants;
a processing system implementing at least one of:

a first algorithm which processes data from the first sensor system to compare a first sensor system result to a threshold level, wherein the threshold level is determined based on one or more of:

i) a result of the determined variation at target locations of the patient's genome,
ii) the first database record, and
iii) the second database record.

and a second algorithm which processes data from the second sensor to determine environmental conditions at the patient's location; and

a feedback device configured to communicate a signal to the patient based on the results of processing by the processing system.

2. The system of example 1 wherein the first sensor system is a non-specific chemical sensor.

3. The system of example 1 wherein the second sensor system is a non-specific chemical sensor.

4. The system of example 3 wherein a plurality of elements in the non-specific chemical sensor respond to an exposure to a chemical in an environment surrounding the non-specific chemical sensor in a pattern of responses which are correlated to a first chemical suspected of being related to an increased risk for developing lung cancer in the patient.

5. The system of example 4 wherein the first chemical is radon.

6. The system of example 4 wherein the first chemical is bis(chloromethyl) ether.

7. The system of example 4 wherein the first chemical is of a family of polycyclic aromatic hydrocarbons.

8. The system of example 3 wherein the non-specific chemical sensor comprises a polymer film doped with chemical species that modulate the absorbent properties of a surface of the polymer film.

9. The system of example 3 wherein the non-specific chemical sensor comprises crystals whose natural oscillation frequency is modulated by absorbance of materials onto surfaces or onto films applied to surfaces of the crystal.

10. The system of example 1 wherein the first sensor system detects biomarkers of lung cancer in the breath of the patient.

11. The system of example 1 wherein the second sensor system is a particulate sensing device.

12. The system of example 1 wherein the second sensor system senses at least a first biological agent.

13. The system of example 12 wherein the biological agent is *Mycobacterium tuberculosis*.

14. The system of example 1 further comprising a stationary sensing system that senses the work environment surrounding the patient while they are at work.

15. The system of example 14 wherein the stationary sensing system senses radioactivity.

16. The system of example 14 wherein the stationary sensing sensor includes chemical exposure sensing.

17. The system of example 14 wherein the stationary sensing system includes particulate exposure sensing.

18. The system of example 14 wherein the stationary sensing system includes blood sensing.

19. The system of example 14 wherein the stationary sensing system includes sensing of a biometric.

20. The system of example 19 wherein the biometric is inhalation rate.

21. The system of example 19 wherein the biometric is the patient's pulse.

22. The system of example 1 wherein the first sensor system that is located within the patient's lung.

23. The system of example 1 wherein the first sensor system that is located within the patient's lung senses chemical markers.

24. The system of example 1 wherein the first sensor system that is located within the patient's lung senses exposure to environmental chemicals.

25. The system of example 1 wherein at least one of the first sensor system and the second sensor system configures its wireless communications devices into a mesh network topology with other regionally distributed sensor systems.

26. A method of reducing a risk of acquiring lung cancer of a patient predisposed to acquiring lung cancer, the method comprising:

determining the predisposition of the patient to acquiring lung cancer by analyzing the patient's genome;
determining the predisposition of the patient to acquiring lung cancer by analyzing the patient's family history;
determining the predisposition of the patient to acquiring lung cancer by analyzing the patient's environment exposure history;
activating a first sensor system, wherein the first sensor system is configured to sense biomarkers related to a cancer state, and wherein the biomarkers emanate from the user;
activating a second sensor system, wherein the second sensor system is configured to sense the local environment of the user for one or more of particulate matter and chemical pollutants;
activating a processing system, wherein the processing system performs a first algorithm which processes stored data originating from the first sensor system and originating from the second sensor system and determines a status to a threshold level, wherein the threshold level is determined at least in part based on the analysis of the patient's genome;
activating a feedback device, wherein the feedback device communicates a signal to the patient based on the status to the alarm level;
monitoring a first environmental status surrounding the patient with the second sensing system;
monitoring a biomarker emanating from the patient with the second sensing device;
detecting a result from one or more of the first sensor system or the second sensor system, wherein the result exceeds the threshold level;
communicating a signal to the patient based on the detected result; and
taking an action to reduce the exposure of the patient to one or more of the first environmental status or the second environmental status.

27. The method according to example 26 wherein the second sensor system is a non-specific chemical sensor.

28. The method according to example 27 wherein a plurality of elements in the non-specific chemical sensor respond to an exposure to a chemical in an environment surrounding the non-specific chemical sensor in a pattern of responses which are correlated to a first chemical suspected of being related to an increased risk for developing lung cancer in the patient.

29. The method according to example 28 wherein the first chemical is asbestos.

30. The method according to example 28 wherein the first chemical is bis(chloromethyl) ether.

31. The method according to example 28 wherein the first chemical is of a family of polycyclic aromatic hydrocarbons.

32. The method according to example 27 wherein the non-specific chemical sensor comprises a polymer film doped with chemical species that modulates the absorbent properties of a surface of the polymer film.

33. The method according to example 27 wherein the non-specific chemical sensor comprises crystals whose natural oscillation frequency is modulated by absorbance of materials onto surfaces or onto films applied to surfaces of the crystal.

34. The method according to example 26 wherein the second sensor system comprises a particulate sensing device.

35. The method according to example 26 wherein the second sensor system senses at least a first biological agent.

36. The method according to example 35 wherein the biological agent is *Mycobacterium tuberculosis*.

37. The method according to example 26 further comprising installing a stationary sensor system that senses the environment of the workplace of the patient.

38. The method according to example 37 wherein the stationary sensor system senses radioactivity.

39. The method according to example 37 wherein the stationary sensor system includes chemical exposure sensing.

40. The method according to example 37 wherein the stationary sensor system includes particulate exposure sensing.

41. The method according to example 37 wherein the stationary sensor system includes blood sensing.

42. The method according to example 37 wherein the stationary sensor system includes sensing of a biometric.

43. The method according to example 42 wherein the biometric is inhalation rate.

44. The method according to example 42 wherein the biometric is the patient's pulse.

45. A system for reducing a potential risk of obtaining cancer, the system comprising:

a means for obtaining genetic data of a patient to determine variation at target locations of the patient's genome, wherein the variations are related to an increased risk of having the form of cancer;
a first sensing device wherein the first sensing device is installed in a location of the home of the patient;
a second sensing device wherein the second sensing device is installed in a location of the work location of the patient;
a processing system, wherein the processing system performs a first algorithm which processes stored data originating from the first sensing device and originating from the second sensing device and determines a status to a threshold level, wherein the threshold level is determined based on a result of the determined variation at target regions of the patient's genome; and
a feedback device, wherein the feedback device communicates a signal to the patient based on the status to the alarm level.

46. A system for detecting a form of cancer, the system comprising:

a means for obtaining genetic data of a patient to determine variation at target locations of the patient's genome, wherein the variations are related to an increased risk of having the form of cancer;
a first database record, wherein the first database record contains records of familial health history;
a second database record, wherein the second database record contains one or more of a null dataset and a record of an environmental exposure of the patient; a first sensor system, wherein the first sensor system is configured to sense biomarkers related to a cancer state, wherein the biomarkers emanate from the user;
a second sensor system, wherein the second sensor system is configured to sense the local environment of the user for one or more of particulate matter and chemical pollutants;
a processing system implementing at least one of:

a first algorithm which processes data from the first sensor system to compare a first sensor system result to a threshold level, wherein the threshold level is determined based on one or more of:

> i) a result of the determined variation at target locations of the patient's genome,
> ii) the first database record, and
> iii) the second database record;

and a second algorithm which processes data from the second sensor to determine environmental conditions at the patient's location;

wherein the first sensor system comprises a local neural network processing device; and
a feedback device configured to communicate a signal to the patient based on the results of processing by the processing system.

**Claims**

1. A system for detecting a form of cancer, the system comprising:

a means for obtaining genetic data of a patient to determine variation at target locations of the patient's genome, wherein the variations are related to an increased risk of having the form of cancer;

a first database record, wherein the first database record contains records of familial health history;
a second database record, wherein the second database record contains one or more of a null dataset and a record of an environmental exposure of the patient;
a first sensor system, wherein the first sensor system is configured to sense biomarkers related to a cancer state, wherein the biomarkers emanate from the user;
a second sensor system, wherein the second sensor system is configured to sense the local environment of the user for one or more of particulate matter and chemical pollutants;
a processing system implementing at least one of:

> a first algorithm which processes data from the first sensor system to compare a first sensor system result to a threshold level, wherein the threshold level is determined based on one or more of:

>> i) a result of the determined variation at target locations of the patient's genome,
>> ii) the first database record, and
>> iii) the second database record.

> and a second algorithm which processes data from the second sensor to determine environmental conditions at the patient's location; and

a feedback device configured to communicate a signal to the patient based on the results of processing by the processing system.

2. A method of reducing a risk of acquiring lung cancer of a patient predisposed to acquiring lung cancer, the method comprising:

determining the predisposition of the patient to acquiring lung cancer by analyzing the patient's genome;
determining the predisposition of the patient to acquiring lung cancer by analyzing the patient's family history;
determining the predisposition of the patient to acquiring lung cancer by analyzing the patient's environment exposure history;
activating a first sensor system, wherein the first sensor system is configured to sense biomarkers related to a cancer state, and wherein the biomarkers emanate from the user;
activating a second sensor system, wherein the second sensor system is configured to sense the local environment of the user for one or more of particulate matter and chemical pollutants;
activating a processing system, wherein the processing system performs a first algorithm which processes stored data originating from the first sensor system and originating from the second sensor system and determines a status to a threshold level, wherein the threshold level is determined at least in part based on the analysis of the patient's genome;

activating a feedback device, wherein the feedback device communicates a signal to the patient based on the status to the alarm level;

monitoring a first environmental status surrounding the patient with the second sensing system;

monitoring a biomarker emanating from the patient with the second sensing device;

detecting a result from one or more of the first sensor system or the second sensor system, wherein the result exceeds the threshold level;

communicating a signal to the patient based on the detected result; and

taking an action to reduce the exposure of the patient to one or more of the first environmental status or the second environmental status.

3. The system of claim 1 wherein the first sensor system is a non-specific chemical sensor; or

wherein the first sensor system detects biomarkers of lung cancer in the breath of the patient; or

wherein the second sensor system senses at least a first biological agent.

4. The system of claim 1 or the method claim 2 wherein the second sensor system is a non-specific chemical sensor.

5. The system or the method of claim 4 wherein a plurality of elements in the non-specific chemical sensor respond to an exposure to a chemical in an environment surrounding the non-specific chemical sensor in a pattern of responses which are correlated to a first chemical suspected of being related to an increased risk for developing lung cancer in the patient; or

wherein the first chemical is radon; or

wherein the first chemical is bis(chloromethyl) ether; or

wherein the first chemical is of a family of polycyclic aromatic hydrocarbons; or

wherein the first chemical is asbestos.

6. The system or the method of claim 4 wherein the non-specific chemical sensor comprises a polymer film doped with chemical species that modulate the absorbent properties of a surface of the polymer film; or

wherein the non-specific chemical sensor comprises crystals whose natural oscillation frequency is modulated by absorbance of materials onto surfaces or onto films applied to surfaces of the crystal.

7. The system of claim 1 or the method of claim 2 wherein the second sensor system comprises a particulate sensing device; or

wherein the second sensor system senses at least a first biological agent, optionally, wherein the biological agent is *Mycobacterium tuberculosis*.

8. The system of claim 1 further comprising a stationary sensing system that senses the work environment surrounding the patient while they are at work.

9. The method according to claim 2 further comprising installing a stationary sensor system that senses the environment of the workplace of the patient.

10. The system of claim 8 or the method of claim 9 wherein the stationary sensing system senses radioactivity; or

wherein the stationary sensing system includes chemical exposure sensing; or

wherein the stationary sensing system includes particulate exposure sensing; or

wherein the stationary sensing system includes blood sensing.

11. The system of claim 8 or the method of claim 9 wherein the stationary sensing system includes sensing of a biometric; wherein, optionally, the biometric is inhalation rate or the patient's pulse.

12. The system of claim 1 wherein the first sensor system that is located within the patient's lung; or

wherein the first sensor system that is located within the patient's lung senses chemical markers; or

wherein the first sensor system that is located within the patient's lung senses exposure to environmental chemicals.

**13.** The system of claim 1 wherein at least one of the first sensor system and the second sensor system configures its wireless communications devices into a mesh network topology with other regionally distributed sensor systems.

**14.** A system for reducing a potential risk of obtaining cancer, the system comprising:

a means for obtaining genetic data of a patient to determine variation at target locations of the patient's genome, wherein the variations are related to an increased risk of having the form of cancer;
a first sensing device wherein the first sensing device is installed in a location of the home of the patient;
a second sensing device wherein the second sensing device is installed in a location of the work location of the patient;
a processing system, wherein the processing system performs a first algorithm which processes stored data originating from the first sensing device and originating from the second sensing device and determines a status to a threshold level, wherein the threshold level is determined based on a result of the determined variation at target regions of the patient's genome; and
a feedback device, wherein the feedback device communicates a signal to the patient based on the status to the alarm level.

**15.** A system for detecting a form of cancer, the system comprising:

a means for obtaining genetic data of a patient to determine variation at target locations of the patient's genome, wherein the variations are related to an increased risk of having the form of cancer;
a first database record, wherein the first database record contains records of familial health history;
a second database record, wherein the second database record contains one or more of a null dataset and a record of an environmental exposure of the patient;
a first sensor system, wherein the first sensor system is configured to sense biomarkers related to a cancer state, wherein the biomarkers emanate from the user;
a second sensor system, wherein the second sensor system is configured to sense the local environment of the user for one or more of particulate matter and chemical pollutants;
a processing system implementing at least one of:

a first algorithm which processes data from the first sensor system to compare a first sensor system result to a threshold level, wherein the threshold level is determined based on one or more of:

i) a result of the determined variation at target locations of the patient's genome,
ii) the first database record, and
iii) the second database record;

and a second algorithm which processes data from the second sensor to determine environmental conditions at the patient's location;

wherein the first sensor system comprises a local neural network processing device; and a feedback device configured to communicate a signal to the patient based on the results of processing by the processing system.

FIG. 1

Fig. 2A

FIG. 2B

Fig. 3A

Fig. 3B

Fig. 4

FIG. 5A

506

509

507

505

504

508

503

501

510                502

500

FIG. 5B

529

530

522                525

520

Fig. 6A

622

621

623

624

614

613

612

631

634

611

632

602 601

633

600

Fig. 6B

660 651 652 680

650

## Fig. 7A

700 770
750
712
722
760
710
720
702
703

FIG. 7B

702 703
730
731
710 720
712 722

FIG. 7C

702 703
730
732
710 720
712 722

FIG. 7D

Fig8E

850

Fig8D

840

Fig8C

830

Fig8A

810

Fig8B

820

910 | Obtain a genetic sample from a patient

920 | Analyze the genetic sample to determine a genetic propensity of the patient to develop lung cancer

930 | Is the genetic propensity of the patient to develop lung cancer high?    931 NO →

940 YES ↓

950 | Monitoring the environment of the patient with one or more of personal monitoring devices, home monitoring devices, and work monitoring devices.

960 | Equipping the patient with a geolocation device that records locations and timestamps for the user which may be married with data in databases of "smart cities" and other regional environmental monitoring systems

970 | Communicating and warning the patient about elevated exposure levels to environmental agents correlated with development of lung cancer in patients.

980 | Taking action to ameliorate the exposure to environmental agents

990 | Increasing scanning and other monitoring of the patient after detected exposures to environmental agents

FIG. 9

1010 | Obtain a genetic sample from a patient

1020 | Analyze the genetic sample to determine a genetic propensity of the patient to develop lung cancer

1030 | Is the genetic propensity of the patient to develop lung cancer elevated?

1031 NO →

1040 YES ↓

1050 | Monitoring the environment of the patient with one or more of home monitoring devices, and work monitoring devices.

1060 | Communicating and warning the patient about elevated exposure levels to environmental agents correlated with development of lung cancer in patients.

1070 | Taking action to ameliorate exposure to the environmental agent.

1080 | Increasing scanning and other monitoring of the patient after detected exposures to environmental agents

FIG. 10

1110 | Obtain a genetic sample from a patient

1120 | Analyze the genetic sample to determine a genetic propensity of the patient to develop lung cancer

1130 | Is the genetic propensity of the patient to develop lung cancer elevated or high?   1131 NO →

1140 YES ↓

1150 | Monitoring one or more of the breath, saliva, and lung tissue of the patient with an analysis device for biomarkers of lung cancer.

1160 | Communicating and warning the patient about levels of biomarkers.

1170 | Increasing scanning and other monitoring of the patient after detected levels of biomarkers.

FIG. 11

Europäisches Patentamt

European Patent Office

Office européen des brevets

# EUROPEAN SEARCH REPORT

Application Number

EP 17 20 2813

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2015/085326 A1 (COLBY BRANDON [US]; KOTWALIWALE ASHWIN [US]) 11 June 2015 (2015-06-11) * paragraphs [0026] - [0028], [0038] - [0056], [0063] - [0064]; figures 1,2 * ----- | 1-15 | INV. G06F19/18 A61B5/00 |
| A | WO 2007/028035 A2 (PROTEUS BIOMEDICAL INC [US]; ZDEBLICK MARK [US]; ROBERTSON TIMOTHY [US] 8 March 2007 (2007-03-08) * page 9, line 33 - page 13, line 15 * ----- | 1-15 | |
| A | BRENDA K. WEIS ET AL: "Personalized Exposure Assessment: Promising Approaches for Human Environmental Health Research", ENVIRONMENTAL HEALTH PERSPECTIVES., vol. 113, no. 7, 3 March 2005 (2005-03-03) , pages 840-848, XP055463115, US ISSN: 0091-6765, DOI: 10.1289/ehp.7651 * the whole document * * ----- | 1-15 | |
| A | WO 2015/051013 A1 (JOAN & IRWIN JACOBS TECHNION CORNELL INNOVATION INST JACOBS INST [US]) 9 April 2015 (2015-04-09) * paragraphs [0073] - [0090], [0112] - [0120] * ----- | 1-15 | TECHNICAL FIELDS SEARCHED (IPC) G06F A61B |

The present search report has been drawn up for all claims

| Place of search | Date of completion of the search | Examiner |
|---|---|---|
| Munich | 9 April 2018 | Zubrzycki, Wojciech |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.....................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 20 2813

This annex lists the patent family members relating to the patent documents cited in the above-mentioned European search report.
The members are as contained in the European Patent Office EDP file on
The European Patent Office is in no way liable for these particulars which are merely given for the purpose of information.

09-04-2018

| Patent document cited in search report | | Publication date | Patent family member(s) | | Publication date |
|---|---|---|---|---|---|
| WO 2015085326 | A1 | 11-06-2015 | AU | 2014360079 A1 | 07-07-2016 |
| | | | CA | 2932914 A1 | 11-06-2015 |
| | | | EP | 3077942 A1 | 12-10-2016 |
| | | | JP | 2017510892 A | 13-04-2017 |
| | | | KR | 20160118391 A | 11-10-2016 |
| | | | SG | 11201604625S A | 28-07-2016 |
| | | | US | 2016321395 A1 | 03-11-2016 |
| | | | WO | 2015085326 A1 | 11-06-2015 |
| WO 2007028035 | A2 | 08-03-2007 | EP | 1920418 A2 | 14-05-2008 |
| | | | JP | 5714210 B2 | 07-05-2015 |
| | | | JP | 2009506838 A | 19-02-2009 |
| | | | JP | 2014176753 A | 25-09-2014 |
| | | | JP | 2016144696 A | 12-08-2016 |
| | | | US | 2008306359 A1 | 11-12-2008 |
| | | | US | 2014051965 A1 | 20-02-2014 |
| | | | WO | 2007028035 A2 | 08-03-2007 |
| WO 2015051013 | A1 | 09-04-2015 | US | 2015102208 A1 | 16-04-2015 |
| | | | WO | 2015051013 A1 | 09-04-2015 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62424719 B
- US 14011902 B
- US 13899528 B